# EUROPEAN PATENT APPLICATION

(11) **EP 4 792 887 A2**
(43) Date of publication of application: **19.08.2026**
(21) Application number: 26189603.9
(22) Date of filing: 31.08.2020
(51) Int. Cl.: A61P 29/00

(54) **PEGLOTICASE FOR TREATMENT OF GOUT IN RENAL TRANSPLANT RECIPIENTS**

(30) Priority: 30.08.2019 US 201962894452 P; 01.06.2020 US 202063033042 P
(62) Document number(s) of the earlier application(s) in accordance with Art. 76 EPC: 20857655.3 / 4 021 482
(71) Applicant: Horizon Therapeutics Ireland DAC, Dublin, A96 F2A8 (IE)
(72) Inventor: PELOSO, Paul, Dublin, D02 E2X4 (IE)
(74) Representative: Lau, Sarah Jane

(57) **Abstract**

The disclosure provides methods of treating gout in renal transplant recipients comprising administering pegloticase as described herein. Also provided are methods of reducing immunogenicity of pegloticase and prolonging the urate lowering effect comprising administration of pegloticase. Also provided are methods of preserving kidney function as a result of administration of the pegloticase.

## Description

### INCORPORATION OF SEQUENCE LISTING

The sequence listing that is contained in the file names "HOR0122101US_ST25," which is 2.84 kilobytes as measured in Microsoft Windows operating system and was created on August 30, 2019, is filed electronically herewith and incorporated herein by reference.

### FIELD OF THE DISCLOSURE

Gout affects approximately 4% of the U.S. population, is the most common form of inflammatory arthritis in men, and is associated with decreased quality of life. The frequency of gout is increasing worldwide, with prevalence rates estimated to be as high as 7% in older men. It is estimated that up to 400,000 people (up to 5% of the estimated 8 million persons with gout) in the United States experience chronic refractory gout, characterized by ongoing symptoms of active disease and a failure to control/maintain serum urate < 6 mg/dL with conventional xanthine oxidase inhibitors (i.e., allopurinol and febuxostat) and uricosuric agents (i.e., probenecid). These patients often have significant, disabling urate deposits in soft tissues and bone known as tophi.

Gout and hyperuricemia (defined as a serum uric acid level > 6.0 mg/dL [Kidney Disease: Improving Global Outcomes {KDIGO} Guidelines, 2009]) are major problems in the population of kidney transplant recipients. The prevalence rate of 13.1% for gout in the kidney transplant recipient population is approximately 12-fold higher than the prevalence rate for gout in the general non-transplanted US population (p <0.0001) (Brigham et al., 2018). Potential contributors to greater disease severity in gout patients with kidney transplants are reduced urate excretion due to renal impairment and reduced utilization of key urate lowering therapies leading to poorer gout control. It is also possible that increased gout severity leads to greater risk of renal failure and hence, the need for transplantation, consistent with evidence of hyperuricemia and gout as risk factors for chronic kidney disease and end-stage renal disease (Radeck et al., 2018). Managing gout in kidney transplant recipients is challenging, as it is complicated by serious underlying diseases and by drug regimens needed to treat these conditions and to prevent graft rejection (Stamp et al., 2005). Some immunosuppressive drugs increase uric acid, and many conventional urate lowering therapies are potentially contraindicated in kidney transplant recipients. For this reason, improved therapies are needed for treating gout in patients who have had kidney transplant.

As described herein in detail, pegloticase (PEGylated uricase; KRYSTEXXA^{®}) is approved as a treatment or therapy for lowering serum uric acid levels. While pegloticase has reportedly been used in a small study involving transplantation patients, the drug was dosed every 3 weeks and not in the approved regimen for those patients (Hershfield et al., 2014). Additionally, Freyne (2018) reported a case of a single cardiac transplant patient who received pegloticase. The present case is believed to be the first report of a study involving renal transplantation patients who are administered pegloticase according to the FDA-approved dosage of IV infusion every 2 weeks.

Thus, in one aspect, the present disclosure provides a method of treating gout in a kidney transplant recipient having a serum uric acid (SUA) level of ≥ 7 mg/dL comprising administering pegloticase to said kidney transplant recipient at a dose of 8 mg every 2 weeks for a total of 12 doses. In some embodiments, a kidney transplant recipient who may benefit from the present disclosure has a serum uric acid (SUA) level of ≥ 6 mg/dL.

In one embodiment, the kidney transplant recipient is administered gout flare prophylaxis before beginning the pegloticase treatment period. In another embodiment, the gout flare prophylaxis comprises colchicine and/or prednisone. In another embodiment, the colchicine is administered at a dose of 0.3 to 0.6 mg/day, and the dose of prednisone is administered at a dose of ≤ 10 mg/day. In another embodiment, colchicine is administered at a dose of 0.3 mg/day for patients with an estimated glomerular filtration rate (eGFR) of ≤ 30 mL/min/1.73m². In another embodiment, prednisone is administered at a dose of ≤ 10 mg/day for at least one week before the first pegloticase treatment in subjects taking cyclosporine. In another embodiment, the gout flare prophylaxis is maintained throughout the pegloticase treatment period.

In another embodiment, a method as described herein further comprises pre-treatment infusion reaction prophylaxis comprising an antihistamine, acetaminophen, and a corticosteroid prior to each treatment with pegloticase. In another embodiment, the pre-treatment prophylaxis comprises:
fexofenadine administered at a dose of 60 or 180 mg orally, taken the day before each treatment with pegloticase;
fexofenadine administered at a dose of 60 or 180 mg orally, and acetaminophen administered at a dose of 1000 mg orally, taken the morning of each treatment with pegloticase; and
methylprednisolone administered at a dose of 125 mg intravenously over a methylprednisolone infusion duration between 10 and 30 minutes, immediately prior to each treatment with pegloticase.

In another embodiment, the dose of fexofenadine administered is 60 mg orally in subjects with decreased renal function and 180 mg orally in subjects with normal renal function. In another embodiment, normal renal function is defined as an estimated glomerular filtration rate (eGFR) of >90 mL/min/1.73m². In another embodiment, the kidney transplant recipient is taking one or more immunosuppressive agent to prevent rejection of the transplanted kidney. In another embodiment, the one or more immunosuppressive agent prevents the formation of anti-drug antibodies against pegloticase. In another embodiment, the prevention of formation of anti-drug antibodies against pegloticase preserves the effectiveness of the pegloticase and decreases the risk of infusion reactions. In another embodiment, a method as described herein further comprises measuring hematology and liver function during treatment. In another embodiment, measuring hematology and liver function comprises measuring one or more of serum uric acid (SUA) levels, eGFR, urine albumin-to-creatinine ratio (UACR), blood pressure, anti-uricase IgG antibodies, and anti-monomethoxypoly(ethyleneglycol) (PEG) antibodies. In another embodiment, the SUA levels of the kidney transplant recipient are determined prior to each dose of pegloticase. In another embodiment, a method as described herein further comprises measuring one or more of trough pegloticase levels, anti-pegloticase antibody levels, and anti-PEG antibody levels, prior to each dose of pegloticase after the first dose. In another embodiment, the SUA level is reduced to less than 6 mg/dL and kidney function is preserved as a result of administration of pegloticase. In some embodiments, the SUA level is reduced to less than 5 mg/dL as a result of administration of the PEGylated uricase. In some embodiments, eGFR remains unchanged or largely unchanged as a result of administration of pegloticase in patients taking one or more immunosuppressive agents. In some embodiments, UACR can be reduced as a result of administration of pegloticase in patients taking one or more immunosuppressive agents. In some specific embodiments, UACR can be reduced by 35% or more as a result of administration of the pegloticase in patients taking one or more immunosuppressive agents. In another embodiment, a method as described herein further comprises measuring one or more of peripheral joint urate deposition volume or bone erosion damage. In another embodiment, the peripheral joint urate deposition volume or bone erosion damage is measured using Dual Energy Computed Tomography (DECT) scan or any advanced imaging technique. In another embodiment, a method as described herein further comprises measuring reduction in tophus size by digital photography. In another embodiment, such a method further comprises evaluating pain assessed by the Health Assessment Questionnaire (HAQ) pain score. In another embodiment, such a method further comprises evaluating the effect of pegloticase on disability assessed by the Health Assessment Questionnaire - Disability Index (HAQ-DI) score. In another embodiment, such a method further comprises evaluating subject-reported changes in physical function and quality of life (QOL).

These and other embodiments of the disclosure are described in detail below.

### BRIEF DESCRIPTION OF THE DRAWINGS

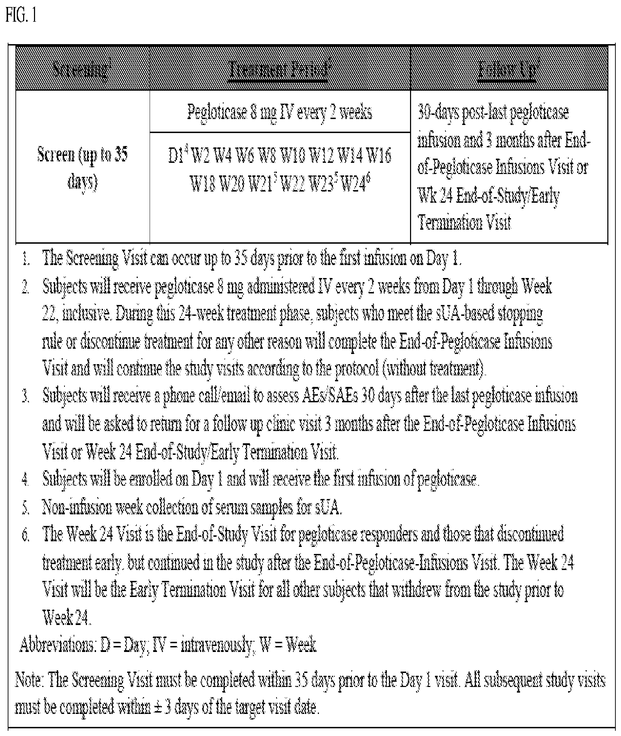

FIG. 1 - Shows a schematic of the study design used in the present disclosure.

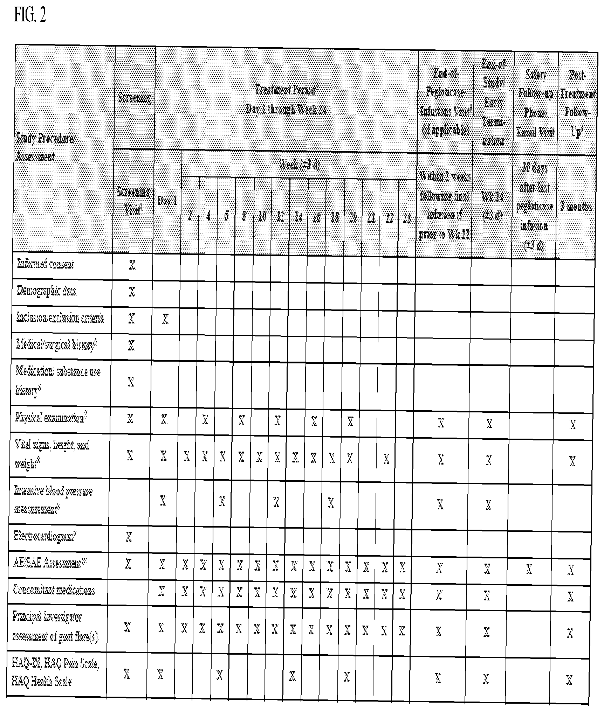

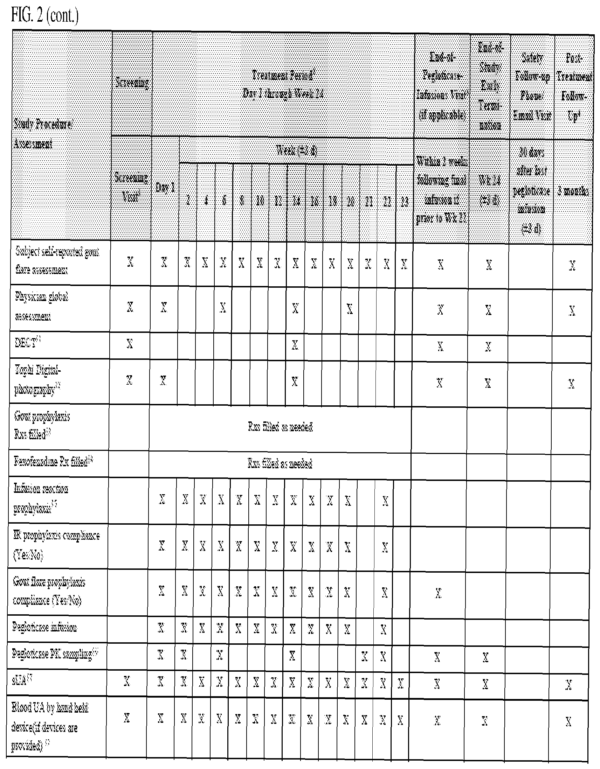

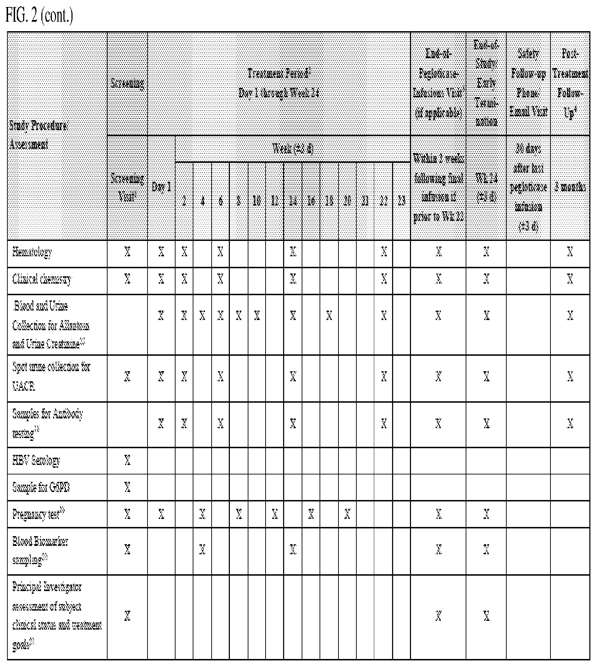

FIG. 2 - Shows the schedule of assessments for administration of pegloticase for treatment of gout in renal transplant patients. ¹The Screening Visit can occur up to 35 days prior to the Day 1 Visit. ²Subjects will be enrolled at the Day 1 Visit to receive pegloticase 8 mg administered IV every 2 weeks from Day 1 through Week 22, inclusive. During this 24-week treatment phase, subjects who meet the sUA based stopping rule or discontinue treatment for any other reason will complete the End-of-Pegloticase-Infusions Visit procedures within 2 weeks and be encouraged to continue to participate in all visits through the end of the study. Subjects will return for regularly scheduled visits and will complete all study visit procedures with the exception of the pegloticase infusion and IR prophylaxis. After the Week 24 Visit (or End-of-Pegloticase-Infusions Visit [if applicable]), subjects should resume regular care for gout per the judgment of the treating physician, including resumption of urate-lowering therapy (ULT) upon pegloticase discontinuation, if appropriate. Subjects will have a 3-month Follow-up visit to assess clinical status, including sUA levels. ³Subjects who end treatment due to the stopping rules or other reasons should complete the End-of-Pegloticase-Infusions Visit within 2 weeks of the last infusion. Subjects should remain on study. ⁴All subjects will have a follow up visit 3 months following the End-of-Pegloticase Infusions Visit or Week 24/End-of-Study/Early Termination Visit. If the subject ends treatment early but remains in the study and the 3-month Post Treatment Follow-up Visit occurs prior to Week 24, the 3-month Post Treatment Follow-up Visit will be adjusted accordingly based on the timing of the last treatment. ⁵The Investigator or designee will collect a complete gout history and other relevant medical/surgical history. ⁶Medication history (i.e., prior medications) will include gout medications, starting at the time of diagnosis and up to (but not including) the Day 1 Visit; substance use history; and all other medications up to (but not including) the Day 1 Visit. Prior concomitant medications (not including gout medications) will be collected for 1 year prior to Screening. ⁷A complete physical examination will be performed at the Screening Visit and will include assessments of head, eyes, ears, nose, and throat (HEENT), heart, lungs, abdomen, skin, extremities, neurological status and musculoskeletal system, and for presence of tophi, as well as gout history and symptom severity. A targeted physical examination (for joint and skin evaluation and assessment of AEs) will be conducted based on potential risk for or occurrence of AEs at Day 1, and prior to administration of infusion at Weeks 4, 8, 12, 16, and 20, the End-of-Pegloticase-Infusions Visit (if applicable), Week 24/End of-Study/Early Termination Visit and 3-month Post-Treatment Follow Up Visit; at a minimum this should include heart, lungs, and abdomen. Clinically significant findings from the targeted physical examinations will be recorded as AEs. ⁸BP, respiratory rate, temperature, and heart rate will be measured at every visit, BEFORE study drug infusion and any scheduled blood draws. Heart rate and BP measurements should be taken after the subject has been in a sitting position and in a rested and calm state with proper positioning including back support, feet flat on the floor, for at least 5 minutes. Subject's arm should be supported at heart level; and the cuff placed on the bare arm. A large cuff should be used as needed to fit the upper arm and the same arm is to be used consistently at each study visit. The Korotkoff phase V will be used to determine DBP. The cuff deflation rate should be 2 mmHg per second. More intensive BP measurements, which include 3 sitting BP measurements, will be collected on Day 1 and at the Week 6, 12, and 18 Visits, the End-of-Pegloticase-Infusions Visit (if applicable) and the 24/End-of-Study/Early Termination Visit. The three blood pressure measurements should be performed at least 2 minutes apart, with BP readings measured to the nearest mm Hg prior to pegloticase infusion. If any of the 3 SBP measurements differ by more than 8 mm Hg or if DBP measurements differ by more than 5 mm Hg, additional sets of 3 sitting BP measurements will be obtained until the measurements differ by less than 8 mm Hg (SBP) and 5 mm Hg (DBP). All values will be recorded in the eCRF. MAP will be calculated from SBP and DBP as MAP = 1/3 * SBP + 2/3 * DBP. Weight should be measured in kilograms or pounds without shoes and recorded at the Screening Visit, prior to pegloticase infusion on Day 1, at the Week 8 and 16 Visits, the non-infusion End-of-Pegloticase-Infusions Visit (if applicable), the Week 24/End-of-Study/Early Termination Visit, and the 3-month Post-Treatment Follow-up Visit. Height will be collected at the Screening Visit only. ⁹Electrocardiogram should be completed during Screening. The electrocardiogram is read at the site. If a subject experiences an AE suspected to be an IR, a 12-lead ECG will also be performed. ¹⁰AEs/SAEs will be collected from the time of signature of the ICF until the 3-month Post-Treatment Follow-up Visit. For each AE, the Investigator will be asked to record if the event was possibly an infusion reaction or anaphylaxis and if so, will be prompted to complete additional eCRFs. ¹¹For sites with DECT capability, the initial DECT scan will be performed after the Screening Visit and prior to the Day 1 Visit. Subsequent DECT scans may be performed ± 7 days from the scheduled visit date. Subjects who end pegloticase infusions prior to Week 24 should follow the scheduled timepoints, but should avoid a repeat DECT scan within 6 weeks of a prior scan (detailed guidance is provided within the Imaging Manual). ¹²Digital photography of hands and feet will be performed according to the instructions provided in the digital photography manual. Other anatomical sites with large tophi may be photographed in addition to the hands and feet at the Investigator's discretion. ¹³Subjects are required to take at least one protocol standard gout flare prophylaxis (i.e., colchicine initial dose 0.3-0.6 mg/day and low-dose prednisone ≤ 10 mg/day); for subjects already taking low dose corticosteroids, colchicine 0.3-0.6 mg/day should be added. In subjects with eGFR ≤ 30 mL/min/1.73m², the dose of colchicine should not be more than 0.3 mg/day. In subjects taking cyclosporine, only low dose prednisone (and no colchicine) should be used for ≥ 1 week before the first infusion. Gout flare prophylaxis should continue for the duration of the 24-week treatment period. Subjects should be monitored closely for symptoms of colchicine excess during the study. Reductions in dose, dose frequency or discontinuation of colchicine are permitted if not tolerated. ¹⁴For IR prophylaxis, fexofenadine (60 - 180 mg orally) will be taken the night before each infusion. ¹⁵IR prophylaxis includes fexofenadine administered the day before each infusion; fexofenadine and acetaminophen (1000 mg orally) administered on the morning of each infusion. In addition, methylprednisolone (125 mg IV from Day 1 to Week 10 and 80 mg IV from Week 12 until the end of treatment) is recommended to be administered over an infusion duration between 10 and 30 minutes, immediately prior to each pegloticase infusion. Alternate IV corticosteroid regimen may be substituted for methylprednisolone at the discretion of the Investigator. The dose of fexofenadine should be 60 mg orally in subjects with decreased renal function and 180 mg orally in subjects with normal renal function (eGFR >90 mL/min/1.73m²). ¹⁶For all subjects, serum samples for pegloticase PK analysis will be collected approximately 15 minutes to 2 hours after the end of infusion on Day 1; prior to infusion and approximately 15 minutes to 2 hours after the end of the infusion at Weeks 2, 6, 14, and 22. An additional PK sample will be collected at Week 21, the End-of-Pegloticase-Infusions Visit (if applicable) and the Week 24/End-of-Study/Early Termination Visit. ¹⁷Serum samples for measurement of sUA levels will be collected at the Screening Visit, within 48 hours prior to each pegloticase infusion and after the end of each pegloticase infusion prior to discharge from the site. Additional serum samples for sUA levels will be collected at non-infusion Visits at Weeks 21 and 23 and the End-of-Pegloticase-Infusions Visit (if applicable), the Week 24/End-of-Study/Early Termination Visit, and the 3-month Follow-up Visit. For subjects whose previous visit's pre-infusion sUA value is ≥ 6 mg/dL, two separate samples/tubes of blood should be collected within 48 hours prior to the pegloticase infusion (except on Day 1 when only 1 pre-infusion sample is required for the central laboratory); one sample/tube will be assessed by the site's local laboratory for pre-infusion sUA results for on-study subject management. If a local laboratory sample is drawn (within 48 hours prior to the pegloticase infusion), a sample for the central laboratory will be drawn prior to the pegloticase infusion on the day of the visit. The second sample/tube will be sent to the central laboratory for analysis and recording in the database. Hand-held uric acid measurement devices may be provided to measure subject uric acid levels in real time. If hand-held devices are provided, blood uric acid levels prior to each pegloticase infusion and after the end of each pegloticase infusion prior to discharge from the site at the selected visits will recorded in database, but NOT to be used to manage pegloticase treatment. ¹⁸Serum samples for evaluation of anti-PEG antibodies and anti-uricase IgG antibodies will be collected prior to the infusion on Day 1 and at Weeks 2, 6, 14, and 22. An additional sample will be collected at each of the End-of-Pegloticase-Infusions Visit (if applicable), the Week 24/End-of-Study/Early Termination Visit, and the 3-month Study Follow Up Visit. In the event of an AE suspected to be an infusion reaction, a serum sample will be collected in a serum separating tube at that time or at the subsequent visit for future evaluation of pegloticase antibodies. ¹⁹For women of childbearing potential, a serum pregnancy test will be performed at the Screening Visit and at the Week 24/End-of-Study/Early Termination Visit. A urine pregnancy test will be performed at all other indicated visits. ²⁰Blood samples will be collected from consenting subjects for serum and for peripheral blood mononuclear cell (PBMC) isolation for potential analysis of inflammatory biomarkers, markers related transplant outcome, gout or gout co-morbidities in response to pegloticase or other potential treatment for gout. Subjects who consent to the optional blood draw for analysis of biomarkers but screen fail will be requested to return 24 weeks +/- 4 weeks for an additional blood sample. Adverse events, concomitant medications and vital signs will also be collected as an unscheduled visit at that time. ²¹The Investigator will review the clinical status and individual subject treatment goals at Screening, the End-of-Pegloticase-Infusions Visit (if applicable) and the Week 24/End-of-Study/Early Termination Visit. ²²Blood and urine samples will be collected prior to pegloticase infusion on Day 1 and at the Week 2, 4, 6, 8, 10, 14, 18, and 22 Visits, the End of Pegloticase Infusions Visit (if applicable), the Week 24/End of Study/Early Termination Visit, and the 3 month Post Treatment Follow-up Visit. In addition, blood and urine samples will be collected after the end of pegloticase infusions prior to discharge from the site at on Day 1, Week 2, 6, 14 and 22 Visits. The urine collected is aliquoted into separate tubes, one of which will be used by the central lab for analysis of creatinine in order to calculate urine allantoin/creatinine ratio. AE = adverse event; d = day(s); DECT = dual-energy computed tomography; G6PD = glucose-6-phosphate dehydrogenase; HAQ = Health Assessment Questionnaire; HAQ-DI = Health Assessment Questionnaire - Disability Index; HBV = hepatitis B virus; IR = infusion reaction; PK = pharmacokinetic; Rx = prescription; SAE = serious adverse event; sUA = serum uric acid; UACR= Urinary Albumin Creatinine Ratio; V = Visit; wk(s) = week(s).

### BRIEF DESCRIPTION OF THE SEQUENCES

SEQ ID NO:1 - Sequence of uricase monomeric subunit of synthetic *Sus scrofa* variant pigKS-ΔN subunit.

### DETAILED DESCRIPTION

### Pegloticase (KRYSTEXXA^{®}, KXX)

Pegloticase (KRYSTEXXA, a recombinant modified mammalian urate oxidase [uricase]), is indicated for treatment-failure gout (TFG) to control hyperuricemia and to manage the signs and symptoms of gout. Pegloticase was granted orphan designation by the FDA on 21 February 2001 (Orphan Drug Act [ODA] #00-1356) and pegloticase 8 mg every 2 weeks was approved by the FDA on 14 September 2010 for the treatment of adult patients with chronic gout refractory to conventional therapy. In view of this, there is a need in the art to develop safer, more convenient, and less immunogenic options for treating hyperuricemia. The invention described herein fulfills this need.

Two replicate pivotal Phase 3 studies for pegloticase were undertaken to establish the efficacy and safety of the product described herein. The primary endpoint was defined as plasma uric acid (pUA) (highly correlated to serum uric acid) reduction to below 6 mg/dL for 80% of the time in Months 3 and 6 combined. The pooled response rate for 8 mg of pegloticase every two weeks was 42%, versus a placebo response rate of 0%. There was also a greater reduction in complete resolution of ≥ 1 tophus in the dosing group receiving pegloticase every 2 weeks, and favorable effect of pegloticase treatment in the reduction of the number of tender and swollen joints. In subsequent open-label extension studies, pegloticase led to continued control of pUA, reduction in gout flares, and continued resolution of tophi, suggesting continuing benefit with extended pegloticase treatment beyond the initial 6 months of therapy, particularly in subjects who met responder criteria in the placebo-controlled trials.

In the Phase 3 pivotal studies, deaths, AEs, and SAEs, as well as laboratory abnormalities were generally equally distributed across placebo and pegloticase treatment groups, with the clear exception of gout flares and infusion reactions (IRs). Pegloticase-treated subjects exhibited a higher rate of gout flares during Months 1-3 as uric acid was being acutely lowered, then a decrease in gout flares vs. placebo during Months 4-6. Despite use of prophylactic medications against hypersensitivity including administration of corticosteroids, antihistamine, and acetaminophen in advance of each pegloticase infusion, IRs were seen in 22/85 (26%) of subjects receiving the 8 mg 2-week regimen. There was no specified definition of anaphylaxis in the Phase 3 protocols, and there were no investigator-reported events of anaphylaxis in the Phase 3 studies with pegloticase.

However, in a post-hoc review applying the National Institute of Allergy and Infectious Diseases/ Food Allergy and Anaphylaxis Network (NIAID/FAAN) criteria (Sampson et al., 2006), it was determined that across the Phase 2 and Phase 3 program, anaphylaxis occurred in 6.5% of subjects treated with pegloticase dosed every 2 weeks. Anaphylaxis generally occurred within 2 hours after treatment. Manifestations included wheezing, peri-oral or lingual edema, or hemodynamic instability, with or without rash or urticaria. All of these events had relatively rapid resolution with the cessation of infusion.

In a post-hoc analysis, the apparent role of immunogenicity in both loss of urate lowering effect and incidence of IRs was appreciated. Only 2% of subjects with anti-pegloticase antibody titers exceeding 1:2430 maintained a urate-lowering response to pegloticase compared with 63% of subjects who were treated for at least 2 months without developing high-titer antibodies (p <0.001) (Sundy et al., 2011). The incidence of IRs was higher among subjects who developed high-titer antibodies compared with those who had titers that did not exceed 1:2430 (60% vs. 19%; p <0.001) (Sundy et al., 2011). In addition, most IRs occurred when sUA levels were greater than 6 mg/dL. Retrospective analyses showed that the loss of urate-lowering efficacy, as reflected by sUA of greater than 6 mg/dL, preceded a patient's first IR, whenever it occurred, in 20 (91%) of 22 subjects treated with pegloticase every 2 weeks.

Therefore, discontinuation of pegloticase therapy in subjects who lose urate-lowering response offers a valuable approach for mitigating the risk of IR. A post-hoc analysis of the biweekly pegloticase dosing group in the randomized controlled trials (RCTs) illustrated the impact of several possible stopping rules based on sUA levels. A stopping rule based on 2 consecutive pre-infusion sUA measurements of greater than 6 mg/dL would have significantly reduced the risk of IR (from 26% to 8%), while having little effect on the treatment response rate (from 42% to 41%) (Keenan et al., 2019). Early post-approval safety surveillance in the US using the stopping rules demonstrated a 69% reduction in the rate of IRs compared with the IR rate recorded during the RCTs (Malamet et al., 2013).

Reducing anti-drug antibodies (ADA) with concomitant administration of the immunomodulatory agent methotrexate (MTX) has been shown to be useful with other infused products that lead to immunogenicity, such as infliximab, in the setting of rheumatoid arthritis treatment. In a recent, prospective, proof-of-concept case series, nine patients with refractory tophaceous gout were pre-treated with MTX prior to co-administration of MTX with KRYSTEXXA. All nine patients were responders as defined by maintaining a serum uric acid at goal of < 6 mg/dL for greater than 80% of the observation period, which ranged from 4.5 to 9 months on therapy. No unexpected adverse effects were reported with this coadministration (Botson and Peterson, 2018).

Inherent in organ transplantation is the need for immunosuppression to maintain the viability of the transplant, and to prevent rejection. There are no formal clinical trials of uricases, including pegloticase, in kidney transplant recipients with gout, although pegloticase has been used successfully in a small number of transplant recipients. Hershfield et al. (2014) reported a trial of pegloticase administered as 8 mg intravenously (IV) every three weeks for up to 5 infusions to 30 patients, including 7 kidney transplant recipients, with refractory gout who were receiving immunosuppression with various combinations of mycophenolate mofetil, cyclosporine, tacrolimus, and azathioprine (AZA). Five of the 7 transplant recipients responded well to pegloticase, and only 1 of 7 developed antibodies to pegloticase, compared to 9 of 20 pegloticase-naive non-transplant recipients who were not on immunosuppressives. Of the two transplant recipients who did not respond well to pegloticase, one discontinued at the time of the second infusion with an unrelated SAE (duodenal ulcer which caused loss of the pancreatic graft), and the other discontinued after the third infusion with an unrelated SAE (myocardial infarction); the subject with the myocardial infarction was also the one transplant recipient who developed anti-pegloticase antibodies.

### Physiochemical Properties of Pegloticase

Pegloticase is a uric acid-specific enzyme that is a monomethoxy-poly(ethylene glycol) (PEG)ylated product (i.e., pegloticase, PEGylated uricase) consisting of recombinant modified mammalian urate oxidase (uricase) produced by a genetically modified strain of *Escherichia coli.* Uricase is covalently conjugated to methoxy-PEG (mPEG) (10 kilodalton [kDa] molecular weight). The complementary deoxyribonucleic acid (cDNA) coding for uricase is based on mammalian sequences. Each uricase subunit has a molecular weight of approximately 34 kDa. The average molecular weight of pegloticase (tetrameric enzyme conjugated to mPEG) is approximately 545 kDa. The physiochemical properties of pegloticase are provided in Table 1.

**Table 1. Physiochemical Properties of Pegloticase**

| | |
|---|---|
| **INN:** | Pegloticase |
| **Chemical Name (INN):** | Oxidase, urate (synthetic *Sus scrofa* variant pigKS-ΔN subunit), homotetramer, amide with α-carboxy-ω-methoxypoly(oxy-1,2-ethanediyl) |
| **National drug code (NDC):** | 75987-080-10 |
| **CAS number:** | 885051-90-1 |
| **Molecular formula:** | CₓH_{y}N₁₆₃₂O_{z}S₃₂ |
| | Wherein, x = ~22,920, y = ~43,095, z = ~10,191 |
| **Molecular weight:** | Monomer pegloticase approximately 545 kDa |
| | (based on the estimation of amino acid sequence of uricase and an average of 10.2 strands of approximately 10 kDa monomethoxypoly(ethylene glycol) (mPEG) per uricase monomeric subunit. The monomethoxypoly(ethylene glycol) strands attached to the uricase protein comprise approximately three-quarters of the molecular weight of pegloticase.) |
| **Chemical Structural Formula:** | {[H3C-O-(CH2CH2-O)m-CO-]n-NH- [TYKKNDEVEFVRTGYGKDMIKVLHIQRDGKYHSIKEVATTVQLTLS SKKDYLHGDNSDVIPTDTIKNTVNVLAKFKGIKSIETFAVTICEHFLSS FKHVIRAQVYVEEVPWKRFEKNGVKHVHAFIYTPTGTHFCEVEQIRN GPPVIHSGIKDLKVLKTTQSGFEGFIKDQFTTLPEVKDRCFATQVYCK WRYHQGRDVDFEATWDTVRSIVLQKFAGPYDKGEYSPSVQKTLYDI QVLTLGQVPEIEDMEISLPNIHYLNIDMSKMGLINKEEVLLPLDNPYG KITGTVKRKLSSRL]}4 (SEQ ID NO:1) |
| | Wherein, m=~225, n=~10.2 and each uricase monomeric subunit has the amino acid sequence listed above. |
| | Approximately 10.2 units of methoxypoly(ethylene glycol) are attached to Lysine(K) residues per uricase monomeric subunit. |
| **Appearance:** | Clear colorless solution, free of visible particles. |

### Safety Pharmacology

Unlike most mammalian species, humans lack the urate oxidase enzymatic pathway for the oxidation and disposition of uric acid and are susceptible to the development of gout. To develop an animal model of hyperuricemia and gout for a therapeutic uricase proof-of-concept study, a mouse was genetically modified by knocking out its endogenous uricase gene (Uox). This genetic lesion results in a marked elevation of plasma uric acid levels, leading to deposition of urate in kidney tissue and causing a profound defect in renal concentrating ability and nephrogenic diabetes insipidus. The studies in the mouse Uox-/- system demonstrate the therapeutic potential of pegloticase administration for the treatment of hyperuricemia and provided a "proof of principle" for the clinical use of pegloticase.

In addition, in nonclinical toxicity studies in which uric acid levels were measured, a decline in uric acid levels following administration of pegloticase (all pegloticase doses associated with these studies) was observed.

The results from the acute and chronic toxicity studies did not indicate any toxic or adverse effect of pegloticase administered with a human exposure 645 times higher than that in the Phase 3 clinical studies (8 mg every 2 weeks) based on the area under the curve values from the 39-week, repeat-dose, dog study (high-dose).

An observation in the chronic toxicology studies is the finding of a dose-dependent increase in vacuolated cells. There were no associated clinical manifestations in any animals in which vacuolated cells were present. Evidence of vacuolated cells, especially in the spleen, has been observed with pegloticase administration in all the chronic toxicity studies as well as the embryo/fetal development and absorption, distribution, metabolism, and excretion studies in the rat. It is thought that vacuolation of spleen macrophages is a result of lysosomal overloading following phagocytosis of persistent circulating macromolecules of high molecular weight. In the 39-week, long-term toxicity studies in dogs, vacuolated cells were also present in the basal area of the lamina propria within the duodenum and jejunum, adrenal cortical cells, hepatic Kupffer cells, and the intimal cells within the aortic outflow area of the heart. The vacuolated cells in the heart and adrenal gland did not stain as macrophages. In the aortic outflow tract of the heart, vacuoles were seen in the cytoplasm of endothelial cells in the intimal lining of the aorta. In the adrenal gland, vacuoles were located within cortical cells in the zona reticularis and zona fasciculata. The clinical significance of these findings and functional consequences are unknown.

### Non-clinical Pharmacokinetics

A series of pharmacokinetic (PK) studies was conducted in rats, rabbits, dogs, and pigs to determine the circulation half-life and bioavailability as a function of the route of pegloticase administration. Plasma pegloticase levels were determined by assaying uricase bioactivity in plasma. As part of the PK studies, antibody levels in plasma were determined 2 weeks after the last injection in the rabbit, dog, and rat. Collectively, the results of the PK studies in these animals lend support to the expectation of high bioavailability and prolonged retention of pegloticase after administration in humans.

Absorption, distribution, metabolism, and excretion of pegloticase were examined in rat studies. Approximately 70% of the dose was excreted in the urine during the course of 7 days after injection.

### Clinical Pharmacokinetics

Pegloticase levels were determined in serum based on measurements of uricase enzyme activity.

Following single IV infusions of 0.5 mg to 12 mg pegloticase in 23 patients with symptomatic gout, maximum serum concentrations of pegloticase increased in proportion to the dose administered.

The PK of pegloticase has not been studied in children and adolescents.

In patients undergoing hemodialysis (Study M0403), pegloticase serum concentrations were not clinically meaningfully affected by 2 hemodialysis sessions. Pre- and post-dialyzer samples, as well as samples taken during dialysis, demonstrated that study drug was not removed by the dialysis process (Bleyer et al., 2015).

No formal studies have been conducted to examine the effects of hepatic impairment on pegloticase PK.

### Risks of Pegloticase

The risks of pegloticase use include: Infusion Reactions (IRs), including anaphylaxis; Hemolysis and methemoglobinemia in patients with glucose-6-phosphate dehydrogenase (G6PD) deficiency; Gout flares; and Congestive heart failure exacerbation.

Subjects with diseases or conditions (e.g., non-compensated congestive heart failure) that could potentially place them at increased risk for these events will be excluded from the study.

It is required that before a subject begins the treatment period, he or she has been taking protocol defined standard gout flare prophylaxis (i.e., colchicine initial dose 0.3 to-0.6 mg/day if tolerated and low-dose prednisone ≤ 10 mg/day); for subjects already taking chronic low dose corticosteroids, colchicine 0.3 to-0.6 mg/day should be added. In subjects with eGFR ≤ 30 mL/min/1.73m², the dose of colchicine should not be more than 0.3 mg/day. In subjects taking cyclosporine, only low dose prednisone (and not colchicine) should be used for ≥ 1 week before the first infusion. Gout flare prophylaxis should continue for the duration of the 24-week treatment period. Subjects should be monitored closely for symptoms of colchicine excess during the study. Reductions in dose, dose frequency or discontinuation of colchicine are permitted if not tolerated.

Since IRs can occur, all subjects will receive pre-treatment prophylaxis consisting of an antihistamine, acetaminophen, and a corticosteroid prior to each infusion of pegloticase. To standardize this regimen, fexofenadine (60 or 180 mg orally) will be taken the day before each infusion; fexofenadine (60 or 180 mg orally) and acetaminophen (1000 mg orally) will be taken the morning of each infusion; and methylprednisolone (125 mg IV from Day 1 to Week 10 and 80 mg IV from Week 12 until the end of treatment) will be administered over a methylprednisolone infusion duration between 10 and 30 minutes, immediately prior to each pegloticase infusion. Alternate IV corticosteroid regimen may be substituted for methylprednisolone at the discretion of the Investigator. The dose of fexofenadine should be 60 mg orally in subjects with decreased renal function and 180 mg orally in subjects with normal renal function (eGFR >90 mL/min/1.73m²).

The risk of anaphylaxis and IRs is higher in patients whose sUA level increases to > 6 mg/dL. Beginning with Week 2, subjects with sUA level ≥ 6 mg/dL at 2 consecutive study visits will be classified as a non-responder. These subjects will discontinue from treatment but remain on study.

In some aspects, a number of different uricase sequences are encompassed herein. The uricase described herein may comprise an amino acid sequence that is at least about 90%, 91%, 92%, 93%, 94%, 95%, 96%, 97%, 98%, or 99% identical to SEQ ID NO:1. In one embodiment, the uricase has the amino acid sequence of SEQ ID NO: 1. In some embodiments, the uricase is at least about 90%, 91%, 92%, 93%, 94%, 95%, 96%, 97%, 98%, or 99% identical to SEQ ID NO: 1. In some aspects, the uricase differs from SEQ ID NO:1 by from about 1 to about 35 amino acids, for example by about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35 amino acids.

In some embodiments, a uricase as described herein comprises a truncation at the N- and/or C-terminus, wherein the truncated uricase retains enzymatic activity. In one embodiment, from about 1-15 (e.g., about 1, 2, 3, 4, 5, 6, 7, 8, 9, 10, 11, 12, 13, 14, or 15) amino acids are truncated from the N-terminus. In another embodiment, from about 1-20 (e.g., about 1, 2, 3, 4, 5, 6, 7, 8, 9, 10, 11, 12, 13, 14, 15, 16, 17, 18, 19, or 20) amino acids are truncated from the C-terminus. In yet another embodiment, from about 1-15 (e.g., about 1, 2, 3, 4, 5, 6, 7, 8, 9, 10, 11, 12, 13, 14, or 15) amino acids are truncated from the N-terminus and from about 1-20 (e.g., about 1, 2, 3, 4, 5, 6, 7, 8, 9, 10, 11, 12, 13, 14, 15, 16, 17, 18, 19, or 20) amino acids are truncated from the C-terminus. In a further embodiment, the aforementioned truncated uricase contains from about 1 to about 14 (e.g., about 1, 2, 3, 4, 5, 6, 7, 8, 9, 10, 11, 12, 13, or 14) additional amino acid changes (e.g., additions, deletions, or substitutions). Methods for assaying enzymatic activity of a uricase are known in the art (e.g., product formation and substrate depletion assays), and any suitable method known in the art can be used to measure the enzymatic activity of the uricases described herein.

It is well understood in the art that processing of expressed proteins can result in the cleavage of the N-terminal methionine residue, a co-translational modification that can occur in both prokaryotic and eukaryotic hosts (Sherman, et al., Bioessays, 3: 27-31 (1985)). This processing, which is enzymatically effected by methionine aminopeptidase, is dependent upon the identity of the amino acid residue adjacent to the amino terminus. Methionine is efficiently removed from proteins when the second residue is glycine or an amino acid with a small side chain such as alanine (Hirel et al., Proc. Natl. Acad. Sci. U.S.A., 86: 8247-8251 (1989) and Huang et al., Biochemistry, 26: 8242-8246 (1987)). However, N-terminal methionine is not cleaved when an amino acid with a large side chain is the adjacent residue. Variable degrees of cleavage may occur when the second residue is an intermediate sized amino acid such as threonine or asparagine (Hirel et al., *supra*). Thus, in some embodiments, the methionine at position 1 of the uricase is cleaved, such that the processed form of the uricase does not contain a methionine at position 1. In another embodiment, the uricase retains the methionine at position 1. In order to prevent cleavage of the methionine at position 1, the uricase may comprise one or more amino acid substitutions or deletions following the N-terminal methionine. Such substitutions or deletions would be designed to result in a large amino acid being at the second position within a sequence. Examples of large amino acids are glutamine, glutamic acid, phenylalanine, methionine, lysine, tyrosine, tryptophan and arginine. In some embodiments, the uricase sequence has been modified to comprise an alanine or other small amino acid at position 2 (i.e., the amino acid next to the N-terminal methionine). Examples of small amino acids are glycine, alanine, serine, proline, threonine, valine and cysteine, but preference is given to the smallest of these (glycine and alanine) to limit the possibility of partial processing (Hirel et al., *supra*).

Short plasma half-life is a problem with most biologics, i.e., they are eliminated from the body by, for example, kidney excretion. Many drugs are eliminated by the kidneys by glomerular filtration based on the size of the drug. However, there are several mechanisms for elimination or inactivation of a catalytically active protein drug from circulation apart from kidney filtration. For example, there may be reduction in activity due to oxidation of amino acid side chains, particularly for an enzyme with oxidase activity such as uricase. Furthermore, inactivation of the enzyme due to proteolysis or, in particular, dissociation of the native tetrameric structure is also possible. One possible explanation for this is that mammalian uricase is a large (tetrameric) enzyme, with a molecular weight of 137 kDa, which should already exceed the generally assumed filtration threshold of the kidneys corresponding to a molecular size around 70 kDa.

As a major family of cell adhesion receptors, integrins are known play a key role in cell-cell and cell-extracellular matrix interactions. The tripeptide Arg-Gly-Asp (RGD) within fibronectin has been shown to mediate cell adhesion through integrin binding. Synthetic peptides containing an RGD motif have been generated specifically to target alpha(v)-integrin for internalization by integrin-dependent endocytosis as a potential cancer therapeutic. Putatively, an integrin binding motif (RGD) could be problematic for a therapeutic that is expected to function in the peripheral blood stream. Thus, in certain aspects of the invention, the uricase does not comprise an RGD sequence.

Methods for mutating amino acids are well-known in the art, and such methods can be used to mutate one or more of the RGD amino acids to any other naturally occurring amino acid. In one embodiment, the arginine in the RGD motif is mutated to a serine, such that the uricase contains an SGD instead of an RGD. In another embodiment, the arginine, the glycine, and/or the aspartic acid in the RGD motif is mutated to any other naturally occurring amino acid, such that the uricase does not contain an RGD motif. In one embodiment, a number of uricase amino acid sequences are aligned using methods known in the art to determine the most highly conserved residue at the amino acid positions where an RGD motif is present, and one or more amino acids present in the RGD motif are mutated to the amino acid residue that is most conserved at that particular amino acid position. For example, if the G and the D of the RGD motif are highly conserved, only the R would be mutated to the amino acid residue that is most highly conserved at that particular position (e.g., serine).

Methods for preparing nucleotide sequences encoding the uricase amino acid sequences disclosed herein are well-known in the art, such that one of ordinary skill in the art can readily prepare a nucleic acid sequence encoding the uricase amino acid sequences disclosed herein. Thus, in one embodiment, the invention comprises a nucleic acid sequence encoding the uricase amino acid sequence disclosed herein. Suitable expression vectors are known and available in the art, such that the invention also encompasses a vector comprising a nucleic acid sequence encoding the uricase amino acid sequence disclosed herein. In yet another embodiment, the invention encompasses a cell line comprising the expression vector. The cell line can be a eukaryotic or a prokaryotic cell line. In a preferred embodiment, the cell line is a prokaryotic cell line, such as *E*. *coli,* Corynebacterium, or *Pseudomonas fluorescens.* In another embodiment, the cell line is a eukaryotic cell line such as *Saccharomyces cerevisiae,* insect cells, etc. Mammalian cell lines such as Chinese hamster ovary (CHO) cells may also be used.

In one embodiment, the invention encompasses a composition comprising the uricase described herein. In one embodiment, a uricase in a composition as described herein is a monomer. In some embodiments, a uricase as described herein may form a dimer, or a trimer, or a tetramer. In other embodiments, a uricase may be modified at the amino (N) end or the carboxy (C) end, or both. In other embodiments, other modifiers deemed appropriate may be used to extend the half-life in circulation in accordance with the present disclosure.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges, and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described. All publications mentioned herein are incorporated herein by reference to disclose and describe the methods and/or materials in connection with which the publications are cited. The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

As used in this specification and the appended claims, the singular forms "a," "an," and "the" include plural referents unless the context clearly dictates otherwise. Thus, for example, "an active agent" refers not only to a single active agent but also to a combination of two or more different active agents, "a dosage form" refers to a combination of dosage forms as well as to a single dosage form, and the like.

Unless defined otherwise, all technical and scientific terms used herein have the meaning commonly understood by one of ordinary skill in the art to which the disclosure pertains. Specific terminology of importance to the description of the present disclosure is defined below.

As used herein, "KRYSTEXXA^{®}" or "KXX" or "pegloticase" or "PEGylated uricase" refers to a covalent conjugate of uricase produced by a genetically modified strain of *Escherichia coli* and monomethoxypoly (ethylene glycol).

As used herein, the term "uricase" or "PEGylated uricase" may be used herein to refer to a PEGylated uricase as described herein. One of skill in the art would understand that many different forms of a uricase may be known and used in accordance with the disclosure, and therefore any PEGylated uricase may be used for treatment of a patient with elevated SUA as described herein. Uricase, as used herein, includes individual subunits, as well as the tetramer, unless otherwise indicated. Mutated uricase, as used herein, refers to uricase molecules having amino acids exchanged with other amino acids.

A conservative mutation, as used herein, is a mutation of one or more amino acids, at or around a position, that does not substantially alter the protein's behavior. In a preferred embodiment, the uricase comprising at least one conservative mutation has the same uricase activity as does uricase without such mutation. In alternate embodiments, the uricase comprising at least one conservative mutation has substantially the same uricase activity, within 5% of the activity, within 10% of the activity, or within 30% of the activity of uricase without such mutation.

Conservative amino acid substitution is defined as a change in the amino acid composition by way of changing amino acids of a peptide, polypeptide or protein, or fragment thereof. In particular embodiments, the uricase has one, two, three or four conservative mutations. The substitution is of amino acids with generally similar properties (e.g., acidic, basic, aromatic, size, positively or negatively charged, polar, non-polar) such that the substitutions do not substantially alter peptide, polypeptide or protein characteristics (e.g., charge, IEF, affinity, avidity, conformation, solubility) or activity.

Truncated uricase, as used herein, refers to uricase molecules having shortened primary amino acid sequences. Amongst the possible truncations are truncations at or around the amino and/or carboxy termini. Specific truncations of this type may be such that the ultimate amino acids (those of the amino and/or carboxy terminus) of the naturally occurring protein are present in the truncated protein. Amino terminal truncations may begin at position 1, 2, 3, 4, 5 or 6. Preferably, the amino terminal truncations begin at position 2, thereby leaving the amino terminal methionine. This methionine may be removed by post-translational modification. In particular embodiments, the amino terminal methionine is removed after the uricase is produced. In a particular embodiment, the methionine is removed by endogenous bacterial aminopeptidase.

A truncated uricase, with respect to the full-length sequence, has one or more amino acid sequences excluded. A protein comprising a truncated uricase may include any amino acid sequence in addition to the truncated uricase sequence, but does not include a protein comprising a uricase sequence containing any additional sequential wild type amino acid sequence. In other words, a protein comprising a truncated uricase wherein the truncation begins at position 6 (i.e., the truncated uricase begins at position 7) does not have, immediately upstream from the truncated uricase, whatever amino acid that the wild type uricase has at position 6.

Unless otherwise indicated by specific reference to another sequence or a particular SEQ ID NO., reference to the numbered positions of the amino acids of the uricases described herein is made with respect to the numbering of the amino acids of the pig uricase sequence. The amino acid sequence of pig uricase and the numbered positions of the amino acids comprising that sequence is set forth in SEQ ID NO:1. As used herein, reference to amino acids or nucleic acids "from position X to position Y" means the contiguous sequence beginning at position X and ending at position Y, including the amino acids or nucleic acids at both positions X and Y.

Uricase genes and proteins have been identified in several mammalian species, for example, pig, baboon, rat, rabbit, mouse, and rhesus monkey. The sequences of various uricase proteins are described herein by reference to their public data base accession numbers, as follows: gi|50403728|sp|P25689; gi|20513634|dbj|BAB91555.1; gil176610 AAA35395.1; gi|20513654|dbj|BAB91557.1; gi|47523606|ref|NP_999435.1; gi|6678509|ref NP_033500.1; gi|57463|emb|CAA31490.1; gi|20|27395|ref|NP_446220.1; gi|137107|sp|P11645; gi|5t458661|ref|XP_497688.1; gi|207619|gb|AAA42318.1; gi|26340770 dbj|BAC34047.1; and gi|57459|emb|CAA30378.1. Each of these sequences and their annotations in the public databases accessible through the National Center for Biotechnology Information (NCBI) is incorporated by reference in its entirety.

### Definitions

Kidney transplant recipient with gout: A patient or subject or participant of the study described herein who previously received a kidney transplant, and who currently has gout or exhibits symptoms of gout, defined as a serum uric acid level of ≥ 6 mg/dL. The term "kidney transplant recipient" may be used synonymously with "patient" or "subject" throughout this disclosure.

Adverse Event (AE): As defined by the ICH, an AE is any untoward medical occurrence in a clinical study subject administered a medicinal (investigational or non-investigational) product, whether or not the event is considered related to the study drug. An AE is therefore any unfavorable and unintended sign (including an abnormal laboratory finding), symptom, or disease (new or exacerbated) temporally associated with the use of the study drug. This includes any occurrence that is new in onset or aggravated in severity or frequency from the baseline condition, or abnormal results of diagnostic procedures, including laboratory test abnormalities.

Examples of an AE include: Conditions newly detected or diagnosed after the signing of the informed consent form (ICF), including conditions that may have been present but undetected prior to the start of the study; Conditions known to have been present prior to the start of the study that worsen after the signing of the ICF; Signs, symptoms, or the clinical sequelae of a suspected drug interaction; and Signs, symptoms, or the clinical sequelae of a suspected overdose of either investigational product or a concomitant medication (overdose per se should not be reported as an AE).

Issues that will not be considered an AE include: Conditions present at the start of the study, should be recorded as medical history; Medical or surgical procedures (e.g., endoscopy, appendectomy; however, a condition that leads to a procedure is an AE if it qualifies according to the definitions above); Situations where an untoward medical occurrence did not occur (e.g., social, diagnostic, elective, or convenience admission to a hospital); Fluctuations of pre-existing disease(s) or condition(s) present or detected at the start of the study that do not represent a clinically significant change from baseline; and Abnormal laboratory or test findings that are not assessed by the Investigator as a clinically significant change from baseline.

AEs of special interest include IRs, anaphylaxis, gout flares, and CV events. AEs of special interest will be recorded. An external adjudication committee will adjudicate the AESIs of IR, anaphylaxis and CV events.

AEs are divided into the categories "serious" and "non-serious." This determines the procedures that must be used to report/document the AE. AE and SAE monitoring will begin from the signature of the ICF until the 3-month Post-Treatment Follow-up Visit. Subjects will be questioned about AEs at each study visit, using nonspecific questions, such as "How have you been feeling since the last study visit?" AEs must be recorded and documented.

Serious Adverse Event (SAE): Based on ICH guidelines, an SAE is any untoward medical occurrence that at any dose: Results in death; Is life threatening; Requires hospitalization or prolongation of existing hospitalization; Results in persistent or significant disability/incapacity; Is a congenital anomaly/birth defect; Is a suspected transmission of any infectious agent via a medicinal product; and Is an important medical event. Any death, life-threatening event, or other SAE experienced by a subject during the course of the study, whether or not judged to be drug-related, must be reported within 24 hours of knowledge of the event by entering the information into the eCRF.

Medical and scientific judgment should be exercised in deciding whether expedited reporting as serious is appropriate in other situations; specifically, important medical events that may not be immediately life threatening or result in death or hospitalization but may jeopardize the subject or may require medical or surgical intervention to prevent one of the other outcomes listed in the above definition. These should usually be considered serious. Examples of such events are invasive cancers, intensive treatment in an emergency room or at home for allergic bronchospasm, blood dyscrasias, or convulsions that do not result in hospitalization, or development of drug dependency or drug abuse.

All AEs, both serious and non-serious, will be assessed for severity using the Rheumatology Common Toxicity Criteria (RCTC) v2.0 (Woodworth et al., 2007). The RCTC v2.0 scale displays Grades 1 through 4 with unique clinical descriptions of severity for each AE (including abnormal laboratory values) based on this general guideline. AEs that do not have a corresponding RCTC term will be assessed for severity based on following criteria: Grade 1 (mild) - asymptomatic or transient, short duration (<1 week), no change in lifestyle, no medication or over-the-counter; Grade 2 (moderate) - symptomatic, duration 1 to 2 weeks, alter lifestyle occasionally, medications give relief (may be prescription), study drug continued; Grade 3 (severe) - prolonged symptoms, reversible, major functional impairment, prescription medications/partial relief, hospitalized <24 hours, temporary study drug discontinuation or/and dose reduced; and Grade 4 (includes life-threatening) - at risk of death, substantial disability, especially if permanent, hospitalized >24 hours, permanent study drug discontinuation.

The relationship of each AE to pegloticase will be determined by the Investigator and the Sponsor based on the following definitions: Not related: There is no plausible temporal relationship or there is another explanation that unequivocally provides a more plausible explanation for the event. Related: There is evidence in favor of a causal relationship (i.e., there is a plausible time course) and ≥ 1 of the following criteria apply: There is a reasonable pharmacological relationship (or known class effect); There is no other more plausible explanation; There is a positive de-challenge (without active treatment of the event); There is a positive re-challenge; and There is a distinguishable dose effect.

The assessment of causality will be based on the information available and may change based upon receipt of additional information. After the initial recording of an AE, the Investigator should proactively follow the subject. Any non-serious AEs that are still ongoing at the end of the study should be reviewed to determine if further follow up is required.

All SAEs should be followed until resolution, until the condition stabilizes, or until the subject is lost to follow-up. Once the SAE is resolved, the corresponding AE eCRF page should be updated.

Disability: A substantial disruption of a person's ability to conduct normal life functions. This definition is not intended to include experiences of relatively minor medical significance, such as uncomplicated headache, nausea, vomiting, diarrhea, influenza, and accidental trauma (e.g., sprained ankle) that may temporarily interfere with or prevent everyday life functions but do not constitute a substantial disruption.

Hospitalization: Refers to a subject who has been admitted to the hospital as an inpatient for any length of time. Treatment on an emergency, outpatient basis for an event not fulfilling any of the definitions of SAEs and not resulting in hospital admission does not qualify for this category. Complications that occur during hospitalization are usually AEs. If a complication prolongs hospitalization or fulfills any other serious criterion, the event will be considered as serious. Hospitalization will not be considered an AE in and of itself. It will be considered an outcome of an AE. Therefore, if there is no associated AE, there is no SAE. For example, hospitalization for elective or pre-planned treatment of a pre-existing condition that did not worsen from baseline will not be considered an AE.

Kidney function parameters: Urine albumin-to-creatinine ratio (UACR) and estimated glomerular filtration rate (eGFR), along with other possible measurements or parameters known in the art. In some embodiments, these parameters may be reduced in patients administered pegloticase, either alone or while taking one or more immunosuppressive agents. In some cases, the UACR may be reduced by 35% or more in some patients, particularly those patients with a baseline albuminuria of greater than 300 mg/g. In some embodiments, eGFR may remain unchanged or largely unchanged in patients administered PEGylated uricase, either alone or while taking one or more immunosuppressive agents.

As used herein, eGFR is calculated by the Modification of Diet in Renal Disease [MDRD] study equation : 175 x (S_{cr[mg/dL]})^{-1.154} x (age)^{-0.203}x (0.742 if female) x (1.212 if African American) or 175 x (S_{cr[µmol/L]}/88.4)^{-1.154} x (age)^{-0.203}x (0.742 if female) x (1.212 if African American.

Unchanged or Largely Unchanged: Refers to a lack of change, or a minimal (i.e., statistically insignificant) change in a measured or observed value. As used herein, "unchanged" or "largely unchanged" may refer to a kidney function parameter (e.g., eGFR and/or UACR) that does not change, or increases or decreases only slightly, as a result of administration of PEGylated uricase, either alone or with one or more immunosuppressive agents.

Life threatening: Refers to an event in which the subject was at risk of death at the time of the event. It does not refer to an event that hypothetically might have caused death if it were more severe, prolonged, or untreated.

Infusion Reaction (IR): Any infusion-related AE or cluster of temporally-related AEs, not attributable to another cause, which occur during the pegloticase infusion and for up to 2 hours post-infusion. Other AEs that occur outside of the 2-hour window following the infusion may also be categorized as an IR. Signs and symptoms of the IR and treatments administered will be documented, and will be adjudicated.

Examples of AEs not considered possible IRs include, but are not limited to: laboratory abnormalities that are unlikely to have occurred during or within 2 hours following the infusion (e.g., anemia), gout flares, most infectious diseases, or the recurrence or worsening of a known chronic medical problem identified in the subject's medical history.

Anaphylaxis: Anaphylaxis will be defined using the NIAID/FAAN criteria (Sampson et al., 2006), and will be adjudicated: (1) Acute onset of an illness (minutes to several hours) with involvement of the skin, mucosal tissue, or both (e.g., generalized hives; pruritus or flushing; urticaria, and angioedema (of lips, tongue, or uvula) and ≥ 1 of the following: Respiratory compromise (e.g., dyspnea, wheeze-bronchospasm, stridor, reduced peak expiratory flow, hypoxemia); and Reduced blood pressure or associated symptoms of end-organ dysfunction (e.g., hypotonia [collapse], syncope, incontinence); (2) Two or more of the following that occur rapidly after exposure to a likely allergen for that subject (minutes to several hours): Involvement of the skin-mucosal tissue (e.g., generalized hives, itch-flush, swollen lips-tongue, uvula); Respiratory compromise (e.g., dyspnea, wheeze-bronchospasm, stridor, reduced peak expiratory flow, hypoxemia); Reduced blood pressure or associated symptoms (e.g., hypotonia [collapse], syncope, incontinence); and Persistent gastrointestinal symptoms (e.g., crampy, abdominal pain, vomiting); (3) Reduced blood pressure after exposure to known allergen for that subject (minutes to several hours): systolic blood pressure <90 mmHg or >30% decrease from that subject's baseline. Any incidence of anaphylaxis should be reported as an SAE.

Gout Flare: It is common for potent urate lowering therapies to lead to acute attacks of gout at the beginning of the treatment. Gout flares will be confirmed through questioning the subject or direct observation by the Investigator and will be recorded as AEs. There is no validated instrument to assess gout flares. Investigator-reported gout flare will be recorded as adverse events. Subjects will also be asked to fill out a subject-reported gout flare questionnaire related to each gout flare, to document subject report of swollen joints, joints that are warm to touch, and level of joint pain (Gaffo et al., 2012) at each visit for exploration of subject-reporting vs. investigator reporting of flares.

Cardiovascular (CV) Events: Cardiovascular AEs will be collected as part of the AE recording. External adjudication will be conducted for selected CV events.

Biopsy Confirmed Acute Rejection: The incidence of renal rejection confirmed by biopsy will be collected as part of the AE recording.

Overdose: A known deliberate or accidental administration of investigational drug, to, or by a study subject, at a dose above that which is assigned to that individual subject according to the study protocol. All cases of medication errors and overdose (with or without associated AEs) will be documented in order to capture this important safety information consistently in the database. AEs associated with an overdose and SAEs of overdose are to be reported. In the event of drug overdose, the subject is to be treated as appropriate.

Pregnancy Reporting: Women of childbearing potential as described above will have a serum pregnancy test at the Screening Visit. Urine pregnancy tests will also be performed at all other time points. Pregnancy will not be considered an AE in this study, however, any pregnancy complications, including an elective termination for medical reasons, should be reported as an AE. Information must be obtained and reported if a female subject suspects that she has become pregnant during the study up to 30 days after the last dose of study treatment. The Investigator will instruct the female subject to stop taking all study drugs. A serum pregnancy test should be performed if any female subject suspects that she has become pregnant during the time frame as defined above. If pregnancy is confirmed, female subject will be withdrawn from the study. Pregnancy will be followed up until the outcome of pregnancy.

Complete pregnancy information, including the outcome of the pregnancy, should be collected in the source documents on the female subject or partner of a male subject. In the absence of complications, follow-up after delivery will be no longer than 8 weeks. Any stillbirths or premature terminations of pregnancies, whether elective, therapeutic, or spontaneous, should be reported on the pregnancy outcome form. Any pregnancy complications, including an elective termination for medical reasons, should be reported as an AE. A spontaneous abortion should always be considered an SAE, as should any congenital defects in the newborn. Any SAE occurring as a result of a post-study pregnancy and considered reasonably related to the investigational product by the Investigator should be reported to the Sponsor. Women who are breastfeeding are not eligible to participate in the study.

Medical History: Refers to medical information for each patient in the study, including gout history (e.g., time of first diagnosis and history of tophi, collected on a gout-specific eCRF) and symptom severity. This information will be conducted at the Screening Visit.

Vital Signs, Height, and Weight: Refers to routine vital signs, including blood pressure, respiratory rate, temperature, and heart rate. Heart rate and BP measurements should be taken after the subject has been in a sitting position and in a rested and calm state with proper positioning including back support and feet flat on the floor for at least 5 minutes. The subject's arm should be supported at heart level; and the cuff placed on the bare arm. A large cuff should be used as needed to fit the upper arm and the same arm is to be used consistently at each study visit. The Korotkoff phase V will be used to determine DBP. The cuff deflation rate should be 2 mm Hg per second. During the pegloticase treatment period study visits, vital signs should be taken before the pegloticase infusion and any time after the end of the infusion, but prior to subject's discharge/release from the site. The same arm and same cuff size should be used. When possible, the same staff member should take all BP measurements for a given subject.

Clinical Laboratory Safety Tests: Refers to the collection of clinical samples for each patient in the study. The following samples may be taken for each patient: Blood (for hematology and clinical chemistry), will be collected at Screening, prior to the pegloticase infusion on Day 1 and at the Week 2, 6, 14, and 22 Visits, the End-of-Pegloticase-Infusions Visit (if applicable), the Week 24/End-of-Study/Early Termination Visit and 3-month Post-Treatment Follow-up Visit. Urine (for albumin:creatinine ratio) samples will be collected at Screening, prior to the pegloticase infusion on Day 1 and at the Week 6, 14, and 22 Visits, the End-of-Pegloticase-Infusions Visit (if applicable) and Week 24/End-of-Study/Early Termination Visit. Urine (for human chorionic gonadotropin) samples will be collected at selected visits.

Safety laboratory assessments will include: Hematology: complete blood count with differential (hemoglobin concentration, hematocrit, erythrocyte count, platelet count, leukocyte count, and differential leukocyte count); Chemistry: albumin, transaminases (aspartate aminotransferase, alanine aminotransferase), alkaline phosphatase, total bilirubin, creatinine (including calculation for eGFR), glucose, sodium, potassium, calcium, chloride, total protein, blood urea nitrogen, and human chorionic gonadotropin (at the Screening Visit and the Week 24/End-of-Study/Early Termination Visit for all female subjects of childbearing potential); and Urine:albumin:creatinine ratio, and human chorionic gonadotropin (for all female subjects of childbearing potential). Safety laboratory samples will be analyzed by the central laboratory. Samples will be collected for analysis at the local laboratory, if needed.

Blood and urine samples will be collected prior to pegloticase infusion on Day 1 and at the Week 2, 4, 6, 8, 10, 14, 18, and 22 Visits, the End-of-Pegloticase-Infusions Visit (if applicable), the Week 24/End-of- Study/Early Termination Visit, and the 3-month Post-Treatment Follow-up Visit. In addition, blood and urine samples will be collected after the end of pegloticase infusions prior to discharge from the site on Day 1, Week 2, 6, 14 and 22 Visits. Both allantoin and creatinine will be analyzed in urine samples collected at these visits to calculate allantoin:creatinine ratio.

Pharmaceutically acceptable: A material that is not biologically or otherwise undesirable, i.e., the material may be incorporated into a pharmaceutical composition administered to a patient without causing any undesirable biological effects or interacting in a deleterious manner with any of the other components of the composition in which it is contained. When the term "pharmaceutically acceptable" is used to refer to a pharmaceutical carrier or excipient, it is implied that the carrier or excipient has met the required standards of toxicological and manufacturing testing or that it is included on the Inactive Ingredient Guide prepared by the U.S. Food and Drug administration. "Pharmacologically active" (or simply "active") as in a "pharmacologically active" (or "active") derivative or analog, refers to a derivative or analog having the same type of pharmacological activity as the parent compound and approximately equivalent in degree. The term "pharmaceutically acceptable salts" include acid addition salts which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Subject or participant or individual or patient: Any patient for whom or which therapy is desired, and generally refers to the recipient of the therapy. In accordance with the present disclosure, a "kidney transplant recipient" may also be used synonymously with these terms.

Treating and treatment: Reduction in severity and/or frequency of symptoms, elimination of symptoms and/or underlying cause, and improvement or remediation of damage. In certain aspects, the terms "treating" and "treatment" as used herein refer to the prevention of the occurrence of symptoms. The phrase "administering to a patient" refers to the process of introducing a composition or dosage form into the patient via an art-recognized means of introduction.

Trough: The lowest concentration of a drug in a patient before the next dose of the drug is administered. For example, trough uricase levels refer to the lowest levels of uricase in a patient before an infusion of uricase. Trough levels may be used by clinicians or practitioners to determine the efficacy of the drug, or the response of the patient to the drug treatment.

### EXAMPLES

Examples of embodiments of the present disclosure are provided in the following examples. The following examples are presented only by way of illustration and to assist one of ordinary skill in using the disclosure. The examples are not intended in any way to otherwise limit the scope of the disclosure.

### Example 1 - Multicenter, Open-Label, Efficacy and Safety Study of Pegloticase in Patients with Uncontrolled Gout who have Undergone Kidney Transplant

Rationale for this Study: During the pre-marketing Phase 3 studies for pegloticase, subjects with organ transplant were excluded from participation; however, subjects with renal impairment were included, and showed comparable safety and efficacy as the general study population. Kidney transplant recipients with uncontrolled gout have the potential to benefit from the ability of pegloticase to rapidly deplete urate deposits, even when urate excretion is impaired. In addition, immunosuppressive agents to prevent rejections have the potential to thwart the formation of ADA against pegloticase, thereby preserving its effectiveness and decreasing the risk of infusion reactions.

This study will test safety and effectiveness of pegloticase 8 mg administered intravenously every 2 weeks in kidney transplant subjects with chronic gout refractory to conventional urate lowering therapy (ULT). Prospective use of sUA stopping rules, with pegloticase treatment cessation (but study continuation) when a subject has a pre-infusion sUA level > 6 mg/dL on two consecutive study visits, will also help confirm the value of this approach to reduce IR risk.

Pegloticase dose selection: The dose of pegloticase and instructions for use are consistent with the current pegloticase prescribing information.

### Objectives

The overall objective of the study is to assess the efficacy and safety of pegloticase in kidney transplant subjects with uncontrolled gout refractory to conventional urate lowering therapy.

The primary objective of this study is to evaluate the effect of pegloticase on the response rate of sustained serum uric acid (sUA) reduction to sUA < 6 mg/dL during Month 6 of treatment.

Secondary objectives of this study include: (1) To evaluate the effect of pegloticase on the response rate of sustained serum uric acid reduction to sUA <5 mg/dL during Month 6 of treatment; (2) To evaluate pain assessed by the Health Assessment Questionnaire (HAQ) pain score; and (3) To evaluate the effect of pegloticase on disability assessed by the Health Assessment Questionnaire - Disability Index (HAQ-DI) score.

Exploratory Objectives of the present study include: (1) To evaluate the effect of pegloticase on the response rate of sustained serum uric acid reduction during Month 3 of treatment and Months 3 and 6 of treatment combined; the uric acid levels over time; the reduction in urate deposition volume and bone erosion due to gout (measured by Dual Energy Computed Tomography [DECT] scan); the reduction in tophus size; complete resolution of at least one tophus by digital photography; the subject-reported changes in physical function and quality of life (QOL); the change in physician assessment of gout; the effect on renal function measured by estimated glomerular filtration rate (eGFR) and urine albumin-to-creatinine ratio (UACR); and the effects on blood pressure (BP); (2) To evaluate the pharmacokinetics (PK) of pegloticase and the profile of anti-monomethoxy-poly(ethylene glycol) (PEG) and anti-uricase IgG antibodies; and (3) To evaluate the safety and efficacy 3 months after the end of treatment with pegloticase.

The Safety Objective of the present study is to assess the overall adverse event (AE)/serious AE (SAE) profile including AEs of special interest (AESI) (infusion reactions [IRs], anaphylaxis, gout flares and cardiovascular [CV] events), laboratory tests, vital signs and physical exam, and incidence of biopsy-confirmed renal rejection.

Safety will be assessed via AE and concomitant medication use monitoring, physical examinations, vital signs, clinical safety laboratory evaluations (hematology, chemistry, urine albumin:creatinine ratio, eGFR), pregnancy testing (if applicable), electrocardiograms (ECGs), and AEs of special interest (i.e., IRs, anaphylaxis, gout flares, and CV events) and incidence of biopsy confirmed renal rejection.

### Study Design

This is a Phase 4, multi-site, open-label, single arm, efficacy and safety study of pegloticase in adult subjects with uncontrolled gout who have undergone kidney transplantation. Twenty subjects will be enrolled.

Because all subjects will receive pegloticase, this study drug will be administered without blinding and all subjects, Investigators and site personnel will know that all subjects are receiving pegloticase.

This study will test safety and effectiveness of pegloticase (administered 8 mg via IV infusion every 2 weeks, consistent with the current pegloticase prescribing information) in kidney transplant subjects with chronic gout refractory to conventional urate lowering therapy. Prospective use of sUA stopping rules, with pegloticase treatment cessation (but study continuation) when a subject has a pre-infusion sUA level > 6 mg/dL on two consecutive study visits, will also help confirm the value of this approach to reduce IR risk.

The study design will include: 1) a Screening Period, which can occur up to 35 days prior to the first infusion on Day 1; 2) a 24-week treatment period which includes an End-of-Study (Week 24)/Early Termination Visit; 3) a safety follow-up phone/email Visit 30 days after the last infusion; and 4) a 3-month post-treatment follow up visit.

During the Screening Period, eligibility will be confirmed.

Samples for measurement of sUA levels will be collected at the Screening Visit, prior to each infusion, 15-60 minutes after the end of each infusion and at the End-of-Study/Early Termination Visit. Additional samples for sUA levels will be collected at non-infusion Visits at Weeks 21 and 23.

Subjects who meet all of the inclusion criteria and none of the exclusion criteria during Screening will be enrolled at the Day 1 Visit to receive pegloticase 8 mg administered IV every 2 weeks from Day 1 through Week 22, inclusive. During this 24-week treatment phase, subjects who meet the stopping rule (pre-dose sUA level > 6 mg/dL at 2 consecutive study visits beginning with the Week 2 Visit) or discontinue treatment for any other reason will complete the End-of-Pegloticase-Infusions Visit procedures within 2 weeks and be encouraged to continue to participate in all visits through the end of the study. Subjects will return for regularly scheduled visits and will complete all study visit procedures with the exception of the pegloticase infusion and IR prophylaxis. After the Week 24 Visit (or End-of-Pegloticase-Infusions Visit [if applicable]), subjects should resume regular care for gout per the judgment of the treating physician, including resumption of urate-lowering therapy (ULT) upon pegloticase discontinuation, if appropriate. Subjects will have a 3-Month Follow-up visit to assess clinical status, including sUA levels.

On days of scheduled infusions, pegloticase will be administered after all pre-dose visit assessments have been completed. The date and start and stop time of infusion will be recorded. The date of the first dose of pegloticase is defined as Day 1. All subsequent doses and study visits will be scheduled based on the Day 1 visit date.

It is required that before a subject begins the treatment period, he or she has been taking protocol defined standard gout flare prophylaxis (colchicine at initial dose of 0.3 to 0.6 mg/day if tolerated and low-dose prednisone ≤ 10 mg/day; for subjects already taking chronic corticosteroids, only colchicine 0.3 to 0.6 mg/day should be added. In subjects with eGFR ≤ 30 mL/min/1.73m², the dose of colchicine should not be more than 0.3 mg/day. In subjects taking cyclosporine, only low dose prednisone (and not colchicine) should be used for ≥ 1 week before the first infusion. Gout flare prophylaxis should continue for the duration of the 24-week treatment period. Subjects should be monitored closely for symptoms of colchicine excess during the study. Reductions in dose, dose frequency or discontinuation of colchicine are permitted if not tolerated.

For Infusion Reaction (IR) prophylaxis, fexofenadine (60 or 180 mg orally) will be taken the day before each infusion; fexofenadine (60 or 180 mg orally) and acetaminophen (1000 mg orally) will be taken in the morning before each infusion; and methylprednisolone (125 mg IV from Day 1 to Week 10 and 80 mg IV from Week 12 until the end of treatment) will be administered over a methylprednisolone infusion duration between 10 and 30 minutes, immediately prior to each pegloticase infusion. Alternate IV corticosteroid regimen may be substituted for methylprednisolone at the discretion of the Investigator. The dose of fexofenadine should be 60 mg orally in subjects with decreased renal function and 180 mg orally in subjects with normal renal function (eGFR >90 mL/min/1.73m²).

Serum samples for PK analysis of pegloticase will be collected approximately 15 minutes to 2 hours after the end of infusion on Day 1, prior to the infusion and approximately 15 minutes to 2 hours after the end of infusion at Weeks 2, 6, 14 and 22. An additional PK sample will be collected at the Week 21 non-infusion visit, End-of-Pegloticase-Infusions Visit (if applicable) and End-of-Study/Early Termination Visit.

Serum samples for evaluation of anti-PEG and anti-uricase immunoglobulin G (IgG) antibodies will be collected prior to the infusion on Day 1 and prior to the infusions at the Weeks 2, 6, 14 and 22. An additional sample will be collected at the End-of-Pegloticase-Infusions Visit (if applicable), End-of-Study/Early Termination Visit and the 3-month follow up visit. In the event of an AE suspected to be an infusion reaction, a sample will be collected at that time or at the subsequent visit for future evaluation of antibodies.

Safety assessments, including monitoring and recording of all AEs, whether or not drug-related, measurement of vital signs, physical examinations, monitoring of hematology and blood chemistry and acute renal rejection, will be performed.

An independent adjudication committee will review reported events of infusion reactions, cardiovascular events and anaphylaxis.

An overview of the study design is provided below and shown in FIG. 1.
1. The Screening Visit can occur up to 35 days prior to the first infusion on Day 1.
   The Screening Visit must be completed within 35 days prior to the Day 1 visit. All subsequent study visits must be completed within ± 3 days of the target visit date.
2. Subjects will receive pegloticase 8 mg administered IV every 2 weeks from Day 1 through Week 22, inclusive. During this 24-week treatment phase, subjects who meet the sUA-based stopping rule or discontinue treatment for any other reason will complete the End-of-Pegloticase Infusions Visit and will continue the study visits according to the protocol (without treatment).
3. Subjects will receive a phone call/email to assess AEs/SAEs 30 days after the last pegloticase infusion and will be asked to return for a follow up clinic visit 3 months after the Week 24/End-of-Study/Early Termination Visit.
4. Subjects will be enrolled on Day 1 and will receive the first infusion of pegloticase.
5. Non-infusion week collection of serum samples for sUA.
6. The Week 24 Visit is the End-of-Study Visit for pegloticase responders and those that discontinued treatment early but continued in the study after the End-of-Pegloticase-Infusions Visit. The Week 24 Visit will be the Early Termination Visit for all other subjects that withdrew from the study prior to Week 24.

### Subject Population:

Subjects eligible for this study will have had a kidney transplant, sUA ≥ 7 mg/dL at Screening and inability to maintain sUA < 6 mg/dL on other urate-lowering therapy or intolerant or contraindicated to conventional urate-lowering therapy, and have clinical evidence of tophaceous deposits or have recurrent gout flare or presence of chronic gouty arthritis. In some embodiments, subjects eligible for this study may have a sUA ≥ 6 mg/dL at Screening.

### Inclusion Criteria:

Eligible subjects must meet/provide all of the following criteria:
1. Willing and able to give informed consent;
2. Willing and able to comply with the prescribed treatment protocol and evaluations for the duration of the study;
3. Adult men or women ≥ 18 years of age;
4. Is a recipient of a de novo kidney from a living or deceased donor and is > 1 year post-transplant prior to screening;
5. Is on a stable standard of care immunosuppression therapy for at least 3 months prior to screening;
6. Kidney allograft is functional at entry, based on an estimated GFR (eGFR) ≥ 15 mL/min/1.73m²;
7. Women of childbearing potential (including those with an onset of menopause < 2 years prior to Screening, non-therapy-induced amenorrhea for < 12 months prior to screening, or not surgically sterile [absence of ovaries and/or uterus]) must have a negative screening serum pregnancy test and will be required to use a medically approved form of birth control during their participation in the study.
8. Uncontrolled gout, defined as:
   A. Hyperuricemia during screening as documented by sUA ≥ 7 mg/dL during Screening and prior to entry into the Treatment Period (Note: the sUA may be repeated up to 3 times during the Screening Period to confirm eligibility), or, in some embodiments, as documented by sUA ≥ 6 mg/dL during Screening, and
   B. Inability to maintain sUA < 6 mg/dL on other urate-lowering therapy or intolerable side effects or contraindicated with conventional urate-lowering therapy, and
   C. At least 1 of the following:
      i. Evidence of tophaceous deposits
      ii. Recurrent gout flares defined as 2 or more flares in the 12 months prior to Screening
      iii. Presence of chronic gouty arthritis; and
9. Able to tolerate low-dose prednisone (<10 mg/day) as part of the required standard gout flare prophylaxis regimen for ≥ 1 week before the first infusion.

### Exclusion Criteria:

Subjects will be ineligible for study participation if they meet any of the following criteria:
1. Any other organ transplant beside kidney;
2. Any severe infection, unless treated and completely resolved at least 2 weeks prior to Day 1;
3. Chronic or active hepatitis B (HBV) infection;
4. Known history of hepatitis C virus RNA positivity unless treated and viral load is negative;
5. Known history of Human Immunodeficiency Virus (HIV) positivity;
6. Glucose-6-phosphate dehydrogenase (G6PD) deficiency (tested at the Screening Visit);
7. Decompensated congestive heart failure or hospitalization for congestive heart failure within 3 months of the Screening Visit, uncontrolled arrhythmia, treatment for acute coronary syndrome (myocardial infarction or unstable angina), or uncontrolled blood pressure (>160/100 mmHg) at the end of the Screening Period (Day 1 prior to infusion);
8. Pregnant, planning to become pregnant, breastfeeding, planning to impregnate female partner, or not using an effective form of birth control, as determined by the Investigator;
9. Prior treatment with pegloticase, another recombinant uricase (rasburicase), or concomitant therapy with a polyethylene glycol-conjugated drug;
10. Known allergy to pegylated products or history of anaphylactic reaction to a recombinant protein or porcine product;
11. Receipt of an investigational drug within 4 weeks or 5 half-lives, whichever is longer, prior to Day 1, or plans to take an investigational drug during the study;
12. Currently receiving systemic or radiologic treatment for ongoing cancer;
13. History of malignancy within 5 years other than non-melanoma skin cancer, in situ carcinoma of cervix, early stage renal cell cancer or early stage prostate cancer that has been completely resected > 2 years prior to screening;
14. Uncontrolled hyperglycemia with a plasma glucose value >240 mg/dL at Screening that is not subsequently controlled by the end of the Screening Period;
15. Diagnosis of osteomyelitis;
16. Known history of hypoxanthine-guanine phosphoribosyl-transferase deficiency, such as Lesch-Nyhan and Kelley-Seegmiller syndrome;
17. Unsuitable candidate for the study, based on the opinion of the Investigator (e.g., cognitive impairment), such that participation might create undue risk to the subject or interfere with the subject's ability to comply with the protocol requirements or complete the study;
18. Currently receiving allopurinol, febuxostat or other urate lowering medications and unable to discontinue medication 7 days prior to Day 1; or
19. Currently receiving probenecid and unable to discontinue medication within 3 days, prior to Day 1.

### Removal of Subjects from Therapy or Study

All subjects are free to withdraw from study participation at any time, for any reason, and without prejudice to their further medical care. In addition, the Investigator may terminate a subject from the study at any time. However, subjects who are removed from pegloticase therapy should remain on study barring withdrawal of consent for study participation.

### Removal of Subjects from Pegloticase Therapy

In addition to completion of therapy through Week 24, the reason for discontinuation from the therapy will be recorded using 1 of the following categories:
Lack of Efficacy: (i.e., sUA level > 6 mg/dL at 2 consecutive study visits beginning with the Week 2 Visit);
Adverse Event: The subject experiences an AE that imposes an unacceptable risk to the subject's health (e.g., anaphylactic reaction), or the subject is unwilling to continue therapy because of an AE. Subjects who discontinue treatment due to an AE should be followed until resolution or stabilization of the AE, or an adequate explanation for the event is obtained;
Physician decision: The Investigator has determined that pegloticase administration poses an unacceptable risk to the subject (specify reason);
Withdrawal by subject (specify reason);
Lost to Follow-up: The subject does not return to the clinic for scheduled assessments, and does not respond to the site's attempts to contact the subject;
Study Terminated by Sponsor: The Sponsor, IRB, or regulatory agency terminates the study;
Pregnancy; and
Death
Study considerations for subjects ending pegloticase infusions prior to 24 weeks
All subjects will remain on study through Week 24 regardless of whether they stop infusions due to sUA stopping rules or other reason (e.g., withdrawal of consent for pegloticase infusions). Subjects are encouraged to continue to participate in all visits through the end of the study. Subjects are especially encouraged to complete study visits at the study site during key efficacy and safety collections at Weeks 20, 21, 22, 23, and 24 so that sUA labs and other key assessments can be completed. During visits between these key efficacy and safety collection visits, in subjects who have stopped infusions, subjects may complete study visits in person or via telephone to collect AEs, concomitant medications and gout flare information.

Activities related to pre/post infusion monitoring or medication dispensation will not be completed once a subject has stopped pegloticase infusions. These activities include: Infusion reaction prophylaxis; IR prophylaxis compliance; Pegloticase infusion; and Pegloticase PK sampling.

Re-introduction of oral ULTs should not start until after the End of Pegloticase Visit laboratory tests are collected

### Post-Treatment Follow-up:

The intent is to obtain at least 3 months of follow-up on each subject after cessation of pegloticase infusions. If the subject ends treatment early but remains in the study and the 3- month Post Treatment Follow-up Visit occurs prior to Week 24, the 3-month Post Treatment Follow-up Visit will be adjusted accordingly based on the timing of the last treatment.

### Removal of Subjects from Study

In addition to completion of therapy and designated study visits through Week 24, the reason for discontinuation from the study will be recorded using 1 of the following categories:
Lost to Follow-up: The subject does not return to the clinic for scheduled assessments, and does not respond to the site's attempts to contact the subject;
Withdrawal by subject: The subject withdraws from the study. The clinical site should attempt to determine the underlying reason for the withdrawal and document it (i.e., voluntary withdraw, relocation, lack of transportation, etc.). The underlying reason should be specified;
Study Terminated by Sponsor: The Sponsor, IRB, or regulatory agency terminates the study; and
Death
In general, subjects that prematurely discontinue from the study for any reason will not be replaced. An exception may be made for subjects who are unevaluable due to the impact of the COVID-19 pandemic and associated restrictions on movement and work. Subjects unable to receive treatment or be evaluated due to restrictions during the COVID-19 pandemic may be replaced, at the discretion of the sponsor. This may result in more subjects than originally planned being enrolled into the study to allow for the originally planned number to be evaluable for the primary efficacy analysis. In addition, a center may be closed and/or replaced for the following administrative reasons: Excessively slow recruitment; or Poor protocol adherence.

### Screen Failures

Subjects who do not meet all of the eligibility criteria or withdraw consent between Screening and Day 1 will be considered screen failures. Screen failures may be allowed to rescreen for the study if both the Investigator and Sponsor are in agreement regarding rescreening and if the Investigator determines that they can satisfy all of the eligibility criteria. Subjects who fail screening but consented to the optional blood draw for biomarker analysis will be requested to return to the site 24 weeks +/- 4 weeks for a second blood sample. Adverse events, concomitant medications, blood samples for hematology and clinical chemistry and vital signs will vital signs will also be collected as an unscheduled visit at that time.

### Dose Regimen/Route of Administration:

Pegloticase 8 mg will be administered via IV infusion over ≥ 120 minutes every 2 weeks for a total of 12 infusions from Day 1 through the Week 22 Visit. The date and start and stop time of each infusion will be recorded. After the Week 24 Visit, subjects should resume regular care for gout per the judgment of the treating physician, including resumption of ULT upon pegloticase discontinuation, if appropriate. Subjects will have a 3-month Follow-up visit to assess clinical status, including sUA levels.

All subjects will receive prophylactic treatment to reduce the risk of acute gout flares beginning ≥ 1 week before the first dose of pegloticase and continuing for the duration of the 24-week treatment period.

Subjects will not be fasting on the day of infusion and will be encouraged to have a snack or normal meal before or after the infusion. Standardized IR prophylaxis consisting of pre-treatment with antihistamines, acetaminophen and corticosteroids will accompany each infusion: fexofenadine (60 or 180 mg orally) will be taken the day before each infusion; fexofenadine (60 or 180 mg orally) and acetaminophen (1000 mg orally) will be taken the morning of each infusion; methylprednisolone (125 mg IV from Day 1 to Week 10 and 80 mg IV from Week 12 until the end of treatment) will be administered over a methylprednisolone infusion duration between 10 and 30 minutes, immediately prior to each pegloticase infusion. Alternate IV corticosteroid regimen may be substituted for methylprednisolone at the discretion of the Investigator. The dose of fexofenadine should be 60 mg orally in subjects with decreased renal function and 180 mg orally in subjects with normal renal function (eGFR >90 mL/min/1.73m²).

All subjects will receive pegloticase at the same dose of 8 mg administered IV every 2 weeks for a total of 12 infusions from Day 1 through the Week 22 Visit. Subjects will not be fasting on the day of infusion and will be encouraged to have a snack or normal meal before or after the infusion. Pre-infusion sUA results must be reported by the local or central laboratory prior to pegloticase infusion.

Standardized IR prophylaxis consisting of pre-treatment with antihistamines, acetaminophen, and corticosteroids will accompany each infusion. The drug name, dose, and timing of these prophylactic medications will be recorded.

Pegloticase will be administered as an admixture of 8 mg in 250 mL of 0.45% or 0.9% Sodium Chloride Injection, USP for IV infusion by gravity feed or infusion pump. Pegloticase will not be administered as an IV push or bolus.

In a patent IV site, the pegloticase preparation will be infused over a period of no less than 120 minutes while the subject is under close observation for any signs of distress. No in-line filter is required; however, if an in-line filter is used, it should be 0.2 µm or larger. At the end of the infusion, the IV line will be flushed with 10 mL of normal saline to ensure the full dose is administered. The date and times of infusion start and stop (inclusive of the IV flush) will be recorded.

### Pegloticase Dose Modifications

Infusion of pegloticase will be immediately interrupted if the subject experiences any significant IR such as respiratory distress, agitation, chest or back pain, urticaria, or another clinically significant event occurring during infusion. If the AE meets the definition of an SAE for IR, the infusion should not be restarted unless the site Investigator determines it is safe to resume the infusion. If the AE does not meet the definition of an SAE for IR, the site Investigator may make the decision to re-start the infusion depending upon the nature and severity of the AE.

Infusions subsequent to an IR in an individual subject may be given in a larger volume of diluent, not to exceed 500 mL. In this case, the infusion duration will also be extended to a minimum of 3 hours. The total volume and duration of infusion will be recorded.

### Dosage Form and Strength Formulation:

Pegloticase is commercially available in the United States as KRYSTEXXA^{®} and will be supplied as clear, colorless, sterile solution in phosphate-buffered saline (PBS), intended for IV infusion after dilution, and will be packaged in sterile, single-use 2-mL glass vials with a Teflon^{®}-coated (latex-free) rubber injection stopper to deliver pegloticase as 8 mg of uricase protein in a 1 mL volume. Pegloticase will be administered as an admixture of 8 mg in 250 mL of 0.45% or 0.9% Sodium Chloride Injection, United States Pharmacopeia (USP) for IV infusion by gravity feed or infusion pump. Pegloticase will not be administered as an IV push or bolus. In the event of an IR, the infusion should be slowed, or stopped, and restarted at a slower rate at the discretion of the Investigator. Infusions subsequent to an IR in an individual subject may be given in a larger volume of diluent, not to exceed 500 mL. In this case, the infusion duration will also be extended to a minimum of 3 hours.

Each mL of pegloticase contains 8 mg of uricase protein conjugated to 24 mg of 10 kDa monomethoxypoly(ethylene glycol). Excipients include disodium hydrogen phosphate dihydrate, sodium chloride, sodium dihydrogen phosphate dihydrate, and water for injection.

Before preparation for use, pegloticase will be stored in the carton, maintained under refrigeration between 2°C and 8°C (36°F and 46°F), protected from light, and will not be shaken or frozen. Pegloticase diluted in infusion bags is stable for 4 hours at 2°C to 8°C (36°F to 46°F) and for 4 hours at room temperature (20°C to 25°C, 68°F to 77°F).

### Preparation and Administration of Pegloticase

Vials of pegloticase will be visually inspected for particulate matter and discoloration before administration, whenever solution and container permit. Vials will not be used if either is present. Using appropriate aseptic technique, 1 mL of pegloticase will be withdrawn from the vial into a sterile syringe. Any unused portion of product remaining in the vial will be discarded. Syringe contents will be injected into a single 250 mL bag of 0.45% or 0.9% Sodium Chloride Injection, USP for IV infusion and will not be mixed or diluted with other drugs. The infusion bag containing the dilute pegloticase solution will be inverted a number of times to ensure thorough mixing but will not be shaken. In accordance with good pharmacy practice, gloves will be worn during preparation of the dose.

The pegloticase infusion must be started within 4 hours of dilution. Before administration, the diluted solution of pegloticase will be allowed to reach room temperature. Pegloticase must never be subjected to artificial heating.

### Duration of Treatment and Follow-up:

Screening: Up to 35 days prior to the Day 1 visit
Treatment Period: Pegloticase for 24 weeks (infusion visits every 2 weeks from Day 1 until the Week 22 Visit).
End-of-Pegloticase-Infusion Visit (if applicable): If the subject discontinues pegloticase treatment prior to the Week 22 infusion, such as due to the sUA stopping rules, the subject will complete this visit within approximately 2 weeks of the last infusion. Subjects will continue in the study.
End-of-Study/Early Termination Visit: Subjects who discontinue treatment during the 24-week treatment period study will be encouraged to continue study participation through the Week 24/End-of-Study Visit. Subjects who discontinue treatment prior to the Week 24 Visit and are not willing to continue in the study will complete the Early Termination Visit.
Safety Follow-up Visit: All subjects will receive a safety follow-up phone call/email approximately 30 days after the last dose of pegloticase to assess if any AEs/SAEs have occurred and will return to the clinic for a follow up visit 3 months after the last pegloticase infusion.

### Criteria for Evaluation:

Efficacy will be assessed by sUA levels, tophus resolution, tophus size, urate deposition volume and bone erosion due to gout (determined by DECT), BP, renal function, gout flare and physician global assessment of gout.

Quality of life will be assessed using the Health Assessment Questionnaire - Disability Index (HAQ-DI), Health Assessment Questionnaire pain scale (HAQ pain scale) and Health Assessment Questionnaire health scale (HAQ health Scale).

The PK of pegloticase and evaluation of anti-PEG and anti-uricase IgG antibodies will be assessed at specified time points.

Safety assessments will include monitoring and recording of all AEs, whether or not drug-related, measurement of vital signs, physical examinations, and monitoring of hematology and blood chemistry and acute renal rejection.

### Hand Held Uric Acid Measurement Device:

Devices may be provided to measure subject uric acid levels in real time.

### Stopping Rules:

### Individual Subject Stopping Rule based on sUA levels:

During the study, all subjects with pre-infusion sUA levels > 6 mg/dL at 2 consecutive study visits beginning with the Week 2 Visit will be discontinued from pegloticase treatment.

### Statistical Analyses:

### Primary Endpoint:

The primary efficacy endpoint is the proportion of Month 6 (Weeks 20, 21, 22, 23 and 24) responders, defined as subjects achieving and maintaining sUA < 6 mg/dL for at least 80% of the time during Month 6.

### Secondary Endpoints:

The secondary endpoints include: (1) The proportion of 5 mg/dL responders during Month 6 (Weeks 20, 21, 22, 23 and 24), defined as subjects achieving and maintaining sUA <5 mg/dL for at least 80% of the time during Month 6; (2) The change from baseline in mean HAQ pain score to Week 24; and (3) The change from baseline in mean HAQ-DI score to Week 24. The HAQ-DI is a self-reported assessment of how the participant's illness affects their ability to function in their daily life over the past week. The HAQ-DI for a participant is calculated as the mean of the following 8 category scores (range: 0 to 3): Dressing and Grooming, Rising, Eating, Walking, Hygiene, Reach, Grip, and Activities. A lower score demonstrates less disability. The disability index is based on the number of domains answered and is computed only if the subject completes answers to at least 6 domains.

The HAQ pain visual analog scale (VAS) is a measure of pain on a continuous 100-point scale. Participants were asked to indicate how much pain they had in the past week as a result of their illness by placing a vertical mark on a horizontal line from 0 (no pain) to 100 (severe pain). A score from 0 to 3 is obtained based on the location of the participant's mark.

### Exploratory Efficacy Endpoints:

During the 24-week Treatment Period, the effect of pegloticase will be evaluated by the following endpoints:
The proportion of Month 3 (Weeks 10, 12, and 14) responders, defined as subjects achieving and maintaining sUA < 6 mg/dL for at least 80% of the time during Month 3;
The proportion of overall responders, defined as subjects achieving and maintaining sUA < 6 mg/dL for at least 80% of the time during Month 3 (Weeks 10, 12, and 14) and Month 6 (Weeks 20, 21, 22, 23 and 24) combined;
The proportion of 5 mg/dL responders during Month 3 (Weeks 10, 12, and 14) and overall (Months 3 and 6 combined) defined as subjects achieving sUA <5 mg/dL for at least 80% of the time during Month 3, and Months 3 and 6, respectively;
The mean change from baseline in sUA to Weeks 14 and 24;
The proportion of subjects with complete resolution of ≥ 1 tophus at Week 24 by digital photography
The proportion of subjects with sUA < 6 mg/dL at Weeks 14 and 24;
The change from baseline in mean urate deposition volume (using DECT scan) to Week 24;
The proportion of subjects who experience a gout flare during each month (Month 1 to 6);
The mean change from baseline in sitting, systolic blood pressure (SBP), diastolic blood pressure (DBP) and mean arterial pressure (MAP) to Week 6, 12, 18 and 24;
The change from baseline in mean tophus size (long axis measured by digital photography) to Week 24 in subjects with tophus present at baseline. The mean change from baseline in bone erosion due to gout (determined by DECT) at Weeks 14 and 24;
The mean change from baseline in eGFR (4 variable-Modification of Diet in Renal Disease [MDRD] formula) to Weeks 6, 12, 18 and 24;
The mean change from baseline in Urinary Albumin Creatinine Ratio (UACR) to Weeks 6, 12, 18 and 24;
The mean change from baseline in HAQ health score to Weeks 14 and 24;
The mean change from baseline in physician global assessment of gout to Weeks 14 and 24; and

### Pharmacokinetic (PK) and Anti-drug Antibody Endpoints:

Serum concentrations of pegloticase; and
Profile of anti-PEG and anti-uricase IgG antibodies.

### Safety and Tolerability Endpoints:

The safety endpoints include the following:
Overall AE/SAE profile: Incidences of AESI: IRs, anaphylaxis, gout flares, cardiovascular events;
Laboratory tests;
Vital signs and physical examination; and
Incidence of renal rejection (biopsy proven).

### Statistical Analysis of Efficacy Parameters

The efficacy analysis will be performed using the modified intention-to-treat (mITT) population, defined as all enrolled subjects who received at least 1 dose of pegloticase. The proportion of Month 6 responders will be summarized, along with a 95% confidence interval (CI) for the proportion. The proportion of 5 mg/dL responders during Month 6 will be analyzed similarly.

A subject will be declared a non-responder if the subject had sUA level ≥ 6 mg/dL at 2 consecutive study visits beginning with the Week 2 Visit through Week 24 (for the primary endpoint) and through Week 14 (for exploratory endpoints). In addition, a subject who withdraws from study treatment for any reason after the first dose of pegloticase and prior to Month 6 (for the primary endpoint) or Month 3 (for exploratory endpoints) will be considered a non-responder if sUA values are not collected at the planned time points.

The proportion of subjects with resolution of ≥ 1 tophus (100% decrease in the area of at least 1 tophus) at Week 24 will be summarized along with the corresponding 95% CI. HAQ scores (Disability Index [DI], pain, and health), sUA, urate volume, tophi size, bone erosion, SBP, DBP, MAP, physician global assessment, eGFR, and UACR will be summarized at each visit with descriptive statistics. Changes from baseline will be summarized along with 95% CIs.

Special consideration may be given to subjects who discontinue the study or miss visits due to reasons related to the COVID-19 pandemic.

### Sample Size Estimate:

A sample size of 20 subjects is planned for this study. The primary efficacy endpoint, the proportion of subjects achieving and maintaining sUA < 6 mg/dL for at least 80% of the time during Month 6, will be demonstrated to be statistically greater than 43.5% (proportion of responders during Month 6 in Phase 3 studies), if at least 14/20 (70%) responders are observed. In that case, the lower bound of a 95% confidence interval for the proportion of responders will be about 46%.

The schedule of assessments is shown in FIG. 2.

### Study Procedures

### Screening Period:

During the Screening Period, study candidates will be evaluated for study entry according to the stated inclusion and exclusion criteria. The procedures described herein will be performed during Screening to establish each candidate's eligibility for enrollment into the study.

### Screening Visit (Within 35 Days of the Day 1 Visit)

Obtain signed, written informed consent. Refusal to provide this consent excludes an individual from eligibility for study participation. Record date informed consent was given and who conducted the process on the appropriate source documentation:
Determine study eligibility through review of the inclusion/exclusion criteria;
Obtain demographic information;
Investigator review of clinical status and subject treatment goals.;
Collect complete gout history (on gout-specific CRF), other relevant medical/surgical history, and medication history, including gout medications starting at the time of diagnosis and up to screening (on gout medications-specific CRF), substance use history, and all other medications currently being taken at Screening;
Perform a complete physical examination, including assessment of HEENT, heart, lungs, abdomen, skin, extremities, neurological status and musculoskeletal including an assessment for the presence of tophi;
Document PI assessment of gout flares;
Record vital signs (BP, respiratory rate, temperature, and heart rate);
Record height and weight;
Perform digital photography of hands and feet;
Perform 12-lead ECG;
Obtain blood samples for hematology and clinical chemistry analysis;
Obtain a serum sample from all females of childbearing potential for performance of a pregnancy test;
Obtain blood samples to evaluate sUA (only 1 sample for central laboratory), G6PD and HBV serology;
Obtain spot urine sample for albumin:creatinine ratio;
Obtain whole blood and serum samples for potential analyses of biomarkers from consenting subjects;
Administer HAQ and record physician global assessments;
Administer subject self-reported gout flare assessment questionnaire;
Inquire about AEs and concomitant medication use; and
For sites with DECT capability, and subjects who provide consent, obtain an optional DECT. DECT will be completed after all eligibility has been confirmed and prior to Day 1.

### Pegloticase Treatment Period

### Day 1

On Day 1, subjects will return to the clinic for the following assessments and the first dose of pegloticase.

Perform a targeted physical examination (at a minimum this should include heart, lungs and abdomen);
Record vital signs (BP, respiratory rate, temperature, and heart rate) prior to the pegloticase infusion and any time after the end of the infusion, but prior to discharge. During the pre-infusion intensive BP collection, three BP measurements should be performed, at least 2 minutes apart, with BP readings measured to the nearest mm Hg prior to pegloticase infusion. If any of the 3 SBP measurements differ by more than 8 mm Hg or if DBP measurements differ by more than 5 mm Hg, a second set of 3 sitting BP measurements will be obtained. All 3 values, as well as the repeat set of 3 measurements if done, will be recorded in the eCRF;
Determine study eligibility through review of the inclusion/exclusion criteria;
Record weight;
Perform digital photography of hands and feet;
Document PI assessment of gout flares;
Administer HAQ and record physician global assessments;
Administer subject self-reported gout flare assessment questionnaire;
Obtain blood samples for hematology and clinical chemistry analysis;
Obtain a urine sample for albumin:creatinine ratio;
Obtain blood and urine samples for allantoin prior to pegloticase infusion and after the end of the infusion prior to discharge from the site;
Obtain a urine sample from all females of childbearing potential for performance of a pregnancy test;
Obtain 1 blood sample for measurement of sUA prior to the pegloticase infusion and after the end of the pegloticase infusion;
If a hand-held device is provided, perform fingerstick for uric acid measurement prior to the pegloticase infusion and after the end of the pegloticase infusion;
Ask Yes/No question regarding gout flare prophylaxis, and IR prophylaxis compliance;
Fill gout prophylaxis, fexofenadine and acetaminophen prescriptions, as needed;
Obtain blood samples for anti-PEG and anti-uricase IgG antibodies prior to the infusion;
Administer IR prophylaxis (i.e., fexofenadine, acetaminophen, and methylprednisolone);
Administer the first dose of pegloticase and record date, volume, and duration of infusion, and start/stop (inclusive of 10 mL flush) times of dosing;
Obtain blood samples for pegloticase PK analysis any time after the end of the infusion, prior to discharge from the site; and
Inquire about AEs and concomitant medication use.

### Week 2

Obtain 1 blood sample to be sent to the central lab for measurement of sUA prior to the pegloticase infusion;
Document PI assessment of gout flares;
Record vital signs (BP, respiratory rate, temperature, and heart rate) prior to the pegloticase infusion and any time after the end of the infusion, but prior to discharge;
Ask Yes/No question regarding gout flare prophylaxis, and IR prophylaxis compliance;
Administer subject self-reported gout flare assessment questionnaire;
Fill gout prophylaxis, fexofenadine and acetaminophen prescriptions, as needed;
Obtain blood samples for hematology and clinical chemistry analysis;
Obtain spot urine sample for albumin:creatinine ratio;
Obtain blood samples for pegloticase PK analysis prior to the pegloticase infusion and any time after the end of the infusion, prior to discharge from the site;
Obtain blood samples for anti-PEG and anti-uricase IgG antibodies prior to the infusion;
Obtain blood and urine samples for allantoin prior to pegloticase infusion and after the end of the infusion prior to discharge from the site;
If hand held device is provided, perform fingerstick for uric acid measurement prior to the pegloticase infusion and after the end of the pegloticase infusion;
Administer IR prophylaxis (i.e., fexofenadine, acetaminophen, and methylprednisolone);
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 4

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA < 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study;
If a hand-held device is provided, perform fingerstick for uric acid measurement prior to the pegloticase infusion and after the end of the pegloticase infusion;
Perform a targeted physical examination (at a minimum this should include heart, lungs and abdomen);
Document PI assessment of gout flares;
Record vital signs (BP, respiratory rate, temperature, and heart rate) prior to the pegloticase infusion and any time after the end of the infusion, but prior to discharge;
Ask Yes/No question regarding gout flare prophylaxis, and IR prophylaxis compliance;
Administer subject self-reported gout flare assessment questionnaire;
Fill gout prophylaxis, fexofenadine and acetaminophen prescriptions, as needed;
Obtain a urine sample from all females of childbearing potential for performance of a pregnancy test;
Obtain whole blood and serum samples for potential analyses of biomarkers from consenting subjects;
Obtain blood and urine samples for allantoin;
Administer IR prophylaxis (i.e., fexofenadine, acetaminophen, and methylprednisolone);
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 6

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA < 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study;
If a hand-held device is provided, perform fingerstick for uric acid measurement prior to the pegloticase infusion and after the end of the pegloticase infusion;
Document PI assessment of gout flares;
Record vital signs (BP, respiratory rate, temperature, and heart rate) prior to the pegloticase infusion and any time after the end of the infusion, but prior to discharge. During the pre-infusion intensive blood pressure collection, three BP measurements should be performed, at least 2 minutes apart, with BP readings measured to the nearest mm Hg prior to pegloticase infusion. If any of the 3 SBP measurements differ by more than 8 mmHg or if DBP measurements differ by more than 5 mm Hg, a second set of 3 sitting blood pressure measurements will be obtained. All 3 values, as well as the repeat set of 3 measurements if done, will be recorded in the eCRF;
Ask Yes/No question regarding gout flare prophylaxis, and IR prophylaxis compliance;
Fill gout prophylaxis, fexofenadine and acetaminophen prescriptions, as needed;
Administer HAQ and record physician global assessments;
Administer subject self-reported gout flare assessment questionnaire;
Obtain blood samples for hematology and clinical chemistry analysis;
Obtain spot urine sample for albumin:creatinine ratio;
Obtain blood and urine samples for allantoin prior to pegloticase infusion and after the end of infusion prior to discharge from the site;
Obtain blood samples for pegloticase PK analysis prior to the pegloticase infusion and any time after the end of the infusion, prior to discharge from the site;
Obtain blood samples for anti-PEG and anti-uricase IgG antibodies prior to the infusion;
Administer IR prophylaxis (i.e., fexofenadine, acetaminophen, and methylprednisolone);
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 8

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA < 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study;
If a hand-held device is provided, perform fingerstick for uric acid measurement prior to the pegloticase infusion and after the end of the pegloticase infusion;
Perform a targeted physical examination (at a minimum this should include heart, lungs and abdomen);
Document PI assessment of gout flares;
Record vital signs (BP, respiratory rate, temperature, and heart rate) prior to the pegloticase infusion and any time after the end of the infusion, but prior to discharge;
Record weight;
Ask Yes/No question regarding gout flare prophylaxis, and IR prophylaxis compliance;
Administer subject self-reported gout flare assessment questionnaire;
Fill gout prophylaxis, fexofenadine and acetaminophen prescriptions, as needed;
Obtain a urine sample from all females of childbearing potential for performance of a pregnancy test;
Obtain blood and urine samples for allantoin;
Administer IR prophylaxis (i.e., fexofenadine, acetaminophen, and methylprednisolone);
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 10

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA ≤ 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study;
If a hand-held device is provided, perform fingerstick for uric acid measurement prior
to the pegloticase infusion and after the end of the pegloticase infusion;
Document PI assessment of gout flares;
Record vital signs (BP, respiratory rate, temperature, and heart rate) prior to the pegloticase infusion and any time after the end of the infusion, but prior to discharge;
Ask Yes/No question regarding gout flare prophylaxis, and IR prophylaxis compliance.
Administer subject self-reported gout flare assessment questionnaire;
Fill gout prophylaxis, fexofenadine and acetaminophen prescriptions, as needed;
Obtain blood and urine samples for allantoin;
Administer IR prophylaxis (i.e., fexofenadine, acetaminophen, and methylprednisolone);
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use;

### Week 12

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA ≤ 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study;
If hand held device is provided, perform fingerstick for uric acid measurement prior to the pegloticase infusion and after the end of the pegloticase infusion;
Perform a targeted physical examination (at a minimum this should include heart, lungs and abdomen);
Document PI assessment of gout flares;
Record vital signs (BP, respiratory rate, temperature, and heart rate) prior to the pegloticase infusion and any time after the end of the infusion, but prior to discharge. During the pre-infusion intensive BP collection, three BP measurements should be performed, at least 2 minutes apart, with BP readings measured to the nearest mm Hg prior to pegloticase infusion. If any of the 3 SBP measurements differ by more than 8 mm Hg or if DBP measurements differ by more than 5 mm Hg, a second set of 3 sitting blood pressure measurements will be obtained. All 3 values, as well as the repeat set of 3 measurements if done, will be recorded in the eCRF;
Ask Yes/No question regarding gout flare prophylaxis, and IR prophylaxis compliance;
Administer subject self-reported gout flare assessment questionnaire;
Fill gout prophylaxis, fexofenadine and acetaminophen prescriptions, as needed;
Obtain a urine sample from all females of childbearing potential for performance of a pregnancy test;
Administer IR prophylaxis (i.e., fexofenadine, acetaminophen, and methylprednisolone);
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 14

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA ≤ 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study;
If a hand-held device is provided, perform fingerstick for uric acid measurement prior to the pegloticase infusion and after the end of the pegloticase infusion;
Document PI assessment of gout flares;
Record vital signs (BP, respiratory rate, temperature, and heart rate) prior to the pegloticase infusion and any time after the end of the infusion, but prior to discharge;
Administer HAQ and record physician global assessments;
Administer subject self-reported gout flare assessment questionnaire;
Perform digital photography of hands and feet;
For sites with DECT capability, and subjects who provide consent, obtain an optional DECT. DECT may be completed within ± 7 days of the scheduled visit;
Ask Yes/No question regarding gout flare prophylaxis, and IR prophylaxis compliance;
Fill gout prophylaxis, fexofenadine and acetaminophen prescriptions, as needed;
Obtain blood samples for hematology and clinical chemistry analysis;
Obtain a urine sample for albumin:creatinine ratio;
Obtain blood and urine samples for allantoin prior to pegloticase infusion and after the end of the infusion prior to discharge from the site;
Obtain blood samples for pegloticase PK analysis prior to the pegloticase infusion and any time after the end of the infusion, prior to discharge from the site;
Obtain blood samples for anti-PEG and anti-uricase IgG antibodies prior to the infusion;
Obtain whole blood and serum samples for potential analyses of biomarkers from consenting subjects;
Administer IR prophylaxis (i.e., fexofenadine, acetaminophen, and methylprednisolone);
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 16

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA ≤ 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study.

If hand held device is provided, perform fingerstick for uric acid measurement prior to the pegloticase infusion and after the end of the pegloticase infusion;
Perform a targeted physical examination (at a minimum this should include heart, lungs and abdomen);
Document PI assessment of gout flares;
Record vital signs (BP, respiratory rate, temperature, and heart rate) prior to the pegloticase infusion and any time after the end of the infusion, but prior to discharge;
Record weight;
Ask Yes/No question regarding gout flare prophylaxis, and IR prophylaxis compliance;
Administer subject self-reported gout flare assessment questionnaire;
Fill gout prophylaxis, fexofenadine and acetaminophen prescriptions, as needed;
Obtain a urine sample from all females of childbearing potential for performance of a pregnancy test;
Administer IR prophylaxis (i.e., fexofenadine, acetaminophen, and methylprednisolone);
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 18

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA ≤ 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study;
If a hand-held device is provided, perform fingerstick for uric acid measurement prior to the pegloticase infusion and after the end of the pegloticase infusion;
Obtain blood and urine samples for allantoin;
Document PI assessment of gout flares;
Record vital signs (BP, respiratory rate, temperature, and heart rate) prior to the pegloticase infusion and any time after the end of the infusion, but prior to discharge. During the pre-infusion intensive BP collection, three BP measurements should be performed, at least 2 minutes apart, with BP readings measured to the nearest mm Hg prior to pegloticase infusion. If any of the 3 SBP measurements differ by more than 8 mm Hg or if DBP measurements differ by more than 5 mm Hg, a second set of 3 sitting blood pressure measurements will be obtained. All 3 values, as well as the repeat set of 3 measurements if done, will be recorded in the eCRF;
Ask Yes/No question regarding gout flare prophylaxis, and IR prophylaxis compliance;
Administer subject self-reported gout flare assessment questionnaire;
Fill gout prophylaxis, fexofenadine and acetaminophen prescriptions, as needed;
Administer IR prophylaxis (i.e., fexofenadine, acetaminophen, and methylprednisolone);
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 20

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA ≤ 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study;
If a hand-held device is provided, perform fingerstick for uric acid measurement prior to the pegloticase infusion and after the end of the pegloticase infusion;
Perform a targeted physical examination (at a minimum this should include heart, lungs and abdomen);
Document PI assessment of gout flares;
Administer HAQ and record physician global assessments;
Administer subject self-reported gout flare assessment questionnaire;
Record vital signs (BP, respiratory rate, temperature, and heart rate) prior to the pegloticase infusion and any time after the end of the infusion, but prior to discharge;
Ask Yes/No question regarding gout flare prophylaxis, and IR prophylaxis compliance;
Fill gout prophylaxis, fexofenadine and acetaminophen prescriptions, as needed;
Obtain a urine sample from all females of childbearing potential for performance of a pregnancy test;
Administer IR prophylaxis (i.e., fexofenadine, acetaminophen, and methylprednisolone).

Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 21

Obtain a blood sample (1 sample) for measurement of sUA;
If hand held device is provided, perform fingerstick for uric acid measurement prior to the pegloticase infusion and after the end of the pegloticase infusion;
Obtain blood samples for pegloticase PK analysis;
Inquire about AEs and concomitant medication use;
Document PI assessment of gout flares;
Administer subject self-reported gout flare assessment questionnaire; and
Fill gout prophylaxis, fexofenadine and acetaminophen prescriptions, as needed;

### Week 22

For subjects with a pre-infusion sUA value of ≥ 6 mg/dL at the previous visit, obtain 1 blood sample for measurement of sUA by the local lab within 48 hours prior to this Visit's pegloticase infusion. Pre-infusion sUA results must be reported by the local laboratory prior to pegloticase infusion. An additional pre-infusion blood sample for measurement of sUA will be drawn on the day of the visit and sent to the central lab. For subjects with pre-infusion sUA ≤ 6 at the previous visit, only the pre-infusion sample on day of visit for central lab is needed.

Stopping rule: Subjects with a pre-infusion sUA level > 6 mg/dL at 2 consecutive study visits beginning with the Week 2 Visit (not including post-infusion samples) will subsequently discontinue treatment and remain in the study;
If a hand-held device is provided, perform fingerstick for uric acid measurement prior to the pegloticase infusion and after the end of the pegloticase infusion;
Document PI assessment of gout flares;
Record vital signs (BP, respiratory rate, temperature, and heart rate) prior to the pegloticase infusion and any time after the end of the infusion, but prior to discharge;
Ask Yes/No question regarding gout flare prophylaxis, and IR prophylaxis compliance;
Administer subject self-reported gout flare assessment questionnaire;
Fill gout prophylaxis, fexofenadine and acetaminophen prescriptions, as needed;
Obtain blood samples for hematology and clinical chemistry analysis;
Obtain blood and urine samples for allantoin prior to pegloticase infusion and after the end of infusion prior to discharge from the site;
Obtain a urine sample for albumin:creatinine ratio;
Administer IR prophylaxis (i.e., fexofenadine, acetaminophen, and methylprednisolone);
Obtain blood samples for pegloticase PK analysis prior to the pegloticase infusion and any time after the end of the infusion, prior to discharge from the site;
Obtain blood samples for anti-PEG and anti-uricase IgG antibodies prior to the infusion;
Administer pegloticase and record date, volume, and duration of infusion, and start/stop (inclusive of 10 mL flush) times of dosing;
Obtain a blood sample (1 sample) for measurement of sUA any time after the end of the infusion, prior to discharge from the site; and
Inquire about AEs and concomitant medication use.

### Week 23

Obtain a blood sample (1 sample) for measurement of sUA;
If a hand-held device is provided, perform fingerstick for uric acid measurement prior to the pegloticase infusion and after the end of the pegloticase infusion;
Inquire about AEs and concomitant medication use;
Document PI assessment of gout flares;
Administer subject self-reported gout flare assessment questionnaire; and
Fill gout prophylaxis, fexofenadine and acetaminophen prescriptions, as needed.

### End-of-Pegloticase-Infusion Visit

Subjects who end pegloticase infusions prior to Week 24 will complete the End-of-Pegloticase-Infusion Visit procedures following their final infusion. Subjects should continue to participate in all visits through the end of the study. Subjects must complete selected study visits at the study site during key efficacy and safety collections at Weeks 12, 14, 20, 21, 22, 23, and 24, so that sUA labs and other key assessments can be completed. During visits between these key efficacy and safety collection visits in subjects who have stopped infusions, subjects may complete study visits in person or via a telephone visit option to collect AEs, conmeds and gout flare information.

The following procedures will be completed at the End-of-Pegloticase-Infusions Visit:
Document PI assessment of gout flares;
Perform a targeted physical examination (at a minimum this should include heart, lungs and abdomen);
Record vital signs (BP, respiratory rate, temperature, and heart rate) prior to the pegloticase infusion and any time after the end of the infusion, but prior to discharge. During the pre-infusion intensive BP collection, three BP measurements should be performed, at least 2 minutes apart, with BP readings measured to the nearest mm Hg prior to pegloticase infusion. If any of the 3 SBP measurements differ by more than 8 mm Hg or if DBP measurements differ by more than 5 mm Hg, a second set of 3 sitting blood pressure measurements will be obtained. All 3 values, as well as the repeat set of 3 measurements if done, will be recorded in the eCRF;
Record weight;
Administer HAQ and record physician global assessments;
Administer subject self-reported gout flare assessment questionnaire;
Investigator review of clinical status and subject treatment goals;
Ask Yes/No question regarding gout flare prophylaxis compliance;
Perform digital photography of hands and feet;
For sites with DECT capability, and subjects who provide consent, obtain an optional DECT. DECT may be completed within ± 5 days of the scheduled visit. Subjects who end pegloticase infusions prior to Week 52 should follow the scheduled timepoints but avoid a repeat DECT scan within 6 weeks of a prior scan.
Obtain a urine sample from all females of childbearing potential for performance of a pregnancy test;
Obtain blood samples for hematology and clinical chemistry analysis;
Obtain a urine sample for albumin:creatinine ratio;
Obtain blood and urine samples for allantoin;
Obtain blood samples for pegloticase PK analysis;
Obtain blood samples for anti-PEG and anti-uricase IgG antibodies;
Obtain whole blood and serum samples for potential analyses of biomarkers from consenting subjects;
Obtain a blood sample (1 sample) for measurement of sUA; and
Inquire about AEs and concomitant medication use.
Week 24/End-of-Study/Early Termination Visit
Document PI assessment of gout flares;
Investigator review of clinical status and subject treatment goals;
Perform a targeted physical examination (at a minimum this should include heart, lungs and abdomen).

Record vital signs (BP, respiratory rate, temperature, and heart rate) prior to the pegloticase infusion and any time after the end of the infusion, but prior to discharge. During the pre-infusion intensive BP collection, three BP measurements should be performed, at least 2 minutes apart, with BP readings measured to the nearest mm Hg prior to pegloticase infusion. If any of the 3 SBP measurements differ by more than 8 mm Hg or if DBP measurements differed by more than 5 mm Hg, a second set of 3 sitting BP measurements will be obtained. All 3 values, as well as the repeat set of 3 measurements if done, will be recorded in the eCRF;
Record weight;
Administer HAQ and record physician global assessments;
Administer subject self-reported gout flare assessment questionnaire;
Perform digital photography of hands and feet;
For sites with DECT capability, and subjects who provide consent, obtain an optional DECT. DECT may be completed within ± 7 days of the scheduled visit;
Obtain a urine sample from all females of childbearing potential for performance of a pregnancy test;
Obtain blood samples for hematology and clinical chemistry analysis;
Obtain blood and urine samples for allantoin;
Obtain blood samples for pegloticase PK analysis;
Obtain blood samples for anti-PEG and anti-uricase IgG antibodies;
Obtain whole blood and serum samples for potential analyses of biomarkers from consenting subjects;
Obtain a blood sample (1 sample) for measurement of sUA; and
If a hand-held device is provided, perform fingerstick for uric acid measurement;
Inquire about AEs and concomitant medication use.

### Safety Follow-up Phone/Email Visits

Thirty (30) days after the last pegloticase infusion, subjects will be contacted by telephone or email to inquire about SAEs.

### Post-Treatment Follow-up

The intent is to obtain at least 3 months of follow-up on each subject after cessation of pegloticase infusions. If the subject ends treatment early but remains in the study and the 3- month Post Treatment Follow-up Visit occurs prior to Week 24, the 3-month Post Treatment Follow-up Visit will be adjusted accordingly based on the timing of the last treatment.

The following procedures will be completed at the 3-month Post-Treatment Follow-up Visit:
Document PI assessment of gout flares;
Record vital signs (blood pressure, respiratory rate, temperature, and heart rate) prior to discharge;
Perform a targeted physical examination (at a minimum this should include heart, lungs and abdomen);
Record weight;
Administer HAQ and record physician global assessments;
Administer subject self-reported gout flare assessment questionnaire;
Perform digital photography of hands and feet;
Obtain blood samples for hematology and clinical chemistry analysis;
Obtain a blood sample (1 sample) for measurement of sUA;
If a hand-held device is provided, perform fingerstick for uric acid measurement prior to the pegloticase infusion and after the end of the pegloticase infusion;
Obtain blood samples for anti-PEG and anti-uricase IgG antibodies (3-month Post-Treatment Follow-up Visit only);
Obtain blood and urine samples for allantoin; and
Inquire about AEs and concomitant medication use.

### Serum Uric Acid

Serum samples for measurement of sUA levels will be collected at the Screening Visit, within 48 hours prior to each pegloticase infusion and after the end of each pegloticase infusion prior to discharge from the site during the pegloticase treatment period on Day 1, at the Week 2, 6, 10, 12, 14, 20 and 22 Visits; and within 48 hours prior to each pegloticase infusion at Weeks 4, 8, 16, and 18. Additional serum samples for sUA levels will be collected at non-infusion Visits at Weeks 21 and 23, the End-of-Pegloticase-Infusions Visit (if applicable), the Week 24/End-of-Study/Early Termination Visit and the 3-month Follow-up Visit. Subjects with an sUA level ≥ 6 mg/dL at 2 consecutive study visits beginning with the Week 2 Visit will discontinue pegloticase and will be encouraged to continue study participation through Week 24.

Two separate samples/tubes of blood should be collected within 48 hours prior to the pegloticase infusion if the previous visit's pre-infusion sUA value is ≥ 6 mg/dL (except on Day 1 when only 1 pre-infusion sample is required for the central laboratory). One sample/tube will be assessed by the site's local laboratory to be used for on-study subject management; pre-infusion sUA results must be reported by the local or central laboratory prior to each pegloticase infusion. If a local laboratory sample is drawn (within 48 hours prior to the pegloticase infusion), a sample for the central laboratory will be drawn prior to the pegloticase infusion on the day of the visit. The second sample/tube will be sent to the central laboratory for analysis and recording in the database.

A subject with a sUA level ≥ 6 mg/dL (based on the local or central laboratory) at 2 consecutive study visits, beginning with the Week 2 Visit, will be discontinued from pegloticase treatment.

Samples that result in discordant results between local and central laboratories will be evaluated and discussed with the Investigator and the Sponsor's Medical Monitor on a case-by-case basis to determine whether the subject should continue on study or discontinue.

Hand-held uric acid measurement devices maybe provided to measure subject uric acid levels in real time. If hand-held devices are provided, blood uric acid levels obtained from the device prior to pegloticase infusion will NOT be used to manage pegloticase treatment.

### Hand-Held Uric Acid Measurement Device

Select sites and patients may be provided with FDA approved devices to measure subject uric acid levels using a hand-held device. Results will be collected pre-infusion and post-infusion and used for exploratory uses only. No in-study treatment decisions will be made based upon the data generated with the hand-held device.

### Physician Global Assessment

The physician global assessment will be collected at Screening and prior to pegloticase infusion at the Day 1 and Week 6, 14, 20, End-of-Pegloticase-Infusions Visit (if applicable), and the Week 24/End-of-Study/Early Termination Visit. The physician will respond to the statement, "Considering the subject's overall health related to gout, rate their gout overall" using a numeric rating scale ranging from 0 (excellent) to 10 (very poor).

For a given subject, if possible, the same qualified Investigator should perform the assessment at each time point.

### Dual-energy Computed Tomography (DECT)

For sites with DECT capability, DECT will be obtained between the Screening Visit (after all eligibility criteria have been confirmed) and the Day 1 Visit, the Week 14 Visit, the End-of-Pegloticase-Infusions Visit (if applicable), and at the Week 24/End-of-Study/Early Termination Visit. The DECT may be completed within ± 7 days of the Week 14 and Week 24 Visits.

Subjects who end pegloticase infusions prior to Week 24 should follow the scheduled timepoints but avoid a repeat DECT scan within 6 weeks of a prior scan (detailed guidance is provided within the imaging manual). Images will be obtained for the hands/wrists and feet/ankles. The imaging will be performed by a study-specific, qualified radiologist.

### Digital Photography

Digital photography of hands and feet will be completed at Screening, Day 1, Week 14, the End-of-Pegloticase-Infusions Visit (if applicable), the Week 24/End-of-Study/Early Termination Visit, and the 3-month Post-Treatment Follow-up Visit. Other anatomical sites with large tophi may be photographed in addition to the hands and feet at the Investigator's discretion.

The sponsor or designee will provide digital photography equipment to each site for photography of the hands and feet.

### Assessment of Individual Tophi Response

All measurable tophi will be measured bi-dimensionally (using the longest diameter and the longest perpendicular to that diameter) and the response of each individual tophus will be categorized according to the change from baseline in area of each tophus at each visit as follows:
Complete Response (CR) - A 100% decrease in the area of the tophus;
Marked Response (MR) - At least a 75% decrease in the area of the tophus;
Partial Response (PR) - At least a 50% decrease in the area of the tophus;
Stable Disease (SD) - Neither a 50% decrease nor a 25% increase in the area of the tophus can be demonstrated;
Progressive Disease (PD) - A 25% or more increase in the area of the tophus; or
Unable to Evaluate (UE) -The tophus cannot be accurately measured for any reason at any given post-baseline time point (e.g., image missing or of poor quality, obvious infection of the tophus).

Each individual unmeasured tophus will be semi-quantitatively assessed based upon the impression of the central reader using the following guideline:
Complete Response - the disappearance of the tophus;
Improved - An approximate 50% or more reduction from baseline in the size of the tophus;
Stable Disease - Neither improvement nor progression from baseline can be determined;
Progressive Disease - An approximate 50% or more increase from baseline in the area of the tophus; or
Unable to Evaluate - The tophus cannot be assessed for any reason at any given post-baseline time point (e.g., image missing or of poor quality, or obvious infection of the tophus).

The overall response for a subject will be based upon the best response among all tophi (including measurable and unmeasurable) for that subject (e.g., if any one tophus shows complete response, the overall response is Complete Response). If any single tophus shows progression, or if a new tophus appears during the study, the overall response for that subject will be Progressive Disease, regardless of the response of any other tophi. New tophi arising outside of the regions photographed at baseline will be captured and reported and will also result in an overall response assessment of Progressive Disease.

### Quality of Life Assessment

The HAQ including the HAQ-DI, pain and health scales, will be administered at Screening and prior to the pegloticase infusion at the Day 1 Visit, the Week 6 and 14 Visit, the End-of-Pegloticase-Infusions Visit (if applicable), the Week 24/End-of-Study/Early Termination Visit, and the 3-month Post-Treatment Follow-up Visit.

The HAQ-DI is a self-reporting functional status instrument that captures the level of pain expedience by the participants in the past week, can be filled out by a subject in less than 5 minutes and requires 1 minute to score.

The index measures disability over the past week by asking a total of 20 questions covering 8 domains of function: dressing and grooming, arising, eating, walking, hygiene, reach, grip, and usual activities. There are at least 2 questions in each domain and the 8 domains represent a comprehensive set of functional activities. The HAQ-DI is calculated by scoring the answer to each question in the HAQ from 0 to 3, with 0 representing the ability to do without any difficulty, and 3 representing inability to do. Any activity that requires assistance from another individual or requires the use of an assistive device raises a 0 or 1 score to a 2. The highest score for each of the 8 domains is summed (range from 0 to 24) and divided by 8 to yield, on a scale with 25 possible values, a Functional Disability Index with a range from 0 to 3. The disability index is based on the number of domains answered and is computed only if the subject completes answers to at least 6 domains (Bruce and Fries, 2003).

The HAQ pain scale consists of a doubly-anchored, horizontal visual analog scale (VAS), that is scored from 0 (no pain) to 100 (severe pain). Subjects are asked to rate the severity of the pain they have had because of illness in the past week by placing a vertical mark on the VAS.

The HAQ health scale consists of a doubly-anchored, horizontal VAS, that is scored from 0 (very well) to 100 (very poor). Subjects are asked to rate how well they are doing, considering all the ways arthritis affects him or her, by placing a vertical mark on the VAS.

### Pharmacokinetic and Anti-drug Antibody Measurements

Serum samples for PK analysis of pegloticase will be collected after the end of infusion on Day 1 (prior to discharge); prior to the pegloticase infusion and after the end of the infusion (prior to discharge) at Weeks 2, 6, 14, and 22. Additional PK samples will be collected at the Week 21 Visit, the End-of-Pegloticase-Infusions Visit (if applicable), and the Week 24/End-of-Study/Early Termination Visit.

Serum samples for evaluation of anti-PEG and anti-uricase IgG antibodies will be collected prior to the pegloticase infusion on Day 1 and at the Week 2, 6, 14, 22 Visits, the End-of-Pegloticase-Infusions Visit (if applicable), the Week 24/End-of-Study/Early Termination Visit, and the 3-month Post-Treatment Follow-up Visit.

Each sample collection date and time will be recorded.

### Gout Flare Prophylaxis

It is required that before a subject begins the treatment period, he or she has been taking protocol defined standard gout flare prophylaxis (i.e., colchicine initial dose 0.3-0.6 mg/day if tolerated and low-dose prednisone ≤ 10 mg/day); for subjects already taking low dose corticosteroids, colchicine 0.3-0.6 mg/day should be added. In subjects with eGFR ≤ 30 mL/min/1.73m², the dose of colchicine should not be more than 0.3 mg/day. In subjects taking cyclosporine, only low dose prednisone (and not colchicine) should be used for ≥ 1 week before the first infusion. Gout flare prophylaxis should continue for the duration of the 24-week treatment period. Subjects should be monitored closely for symptoms of colchicine excess during the study. Reductions in dose, dose frequency or discontinuation of colchicine are permitted if not tolerated.

Prescriptions are to be filled at a local pharmacy, as needed. At study visits, the subject will be asked a Yes/No question whether gout flare prophylaxis was taken per protocol.

### Gout Flare Treatment

An increase in gout flares is frequently observed upon initiation of ULT, including pegloticase. Subjects will be instructed to contact the site within 12 hours of the onset of symptoms. Gout flares will be confirmed through questioning or direct observation, as detailed herein elsewhere. All subjects who experience a gout flare during the study will be prescribed anti-inflammatory treatment (e.g., corticosteroids, colchicine, and intra-articular steroid injections), as is clinically indicated or deemed necessary on an individual basis at the discretion of the investigator (Becker, 2019). Pain medications for gout flare should be administered according to standard of care as is clinically indicated or deemed necessary on an individual basis at the discretion of the investigator. All medications will be documented.

Colchicine can be prescribed. The precise dose and regimen of colchicine should be individualized for each subject based on renal function and gastrointestinal intolerance by the Investigator and documented on the concomitant medication eCRF.

### Infusion Reaction Prophylaxis

Since IRs can occur with pegloticase, all subjects will receive IR prophylaxis prior to each infusion, consisting of an antihistamine, acetaminophen, and a corticosteroid. To standardize this regimen, subjects will receive fexofenadine (60 or 180 mg orally) the day before each infusion; fexofenadine (60 or 180 mg orally) and acetaminophen (1000 mg orally) the morning of each infusion; and methylprednisolone (125 mg IV from Day 1 to Week 10 and 80 mg IV from Week 12 until the end of treatment) will be administered over a methylprednisolone infusion duration between 10 and 30 minutes, immediately prior to each pegloticase infusion. Alternate IV corticosteroid regimen may be substituted for methylprednisolone at the discretion of the Investigator. The dose of fexofenadine should be 60 mg orally in subjects with decreased renal function and 180 mg orally in subjects with normal renal function (eGFR >90 mL/min/1.73m²). Substitution of the corticosteroid is not allowed. The name, dose, route, date, and time of administration of each prophylactic medication will be recorded in the medical record and in the eCRF. The methylprednisolone used for IR prophylaxis will be supplied by the site. Other IR medications administered prior to each infusion may also be supplied by the site.

Prescriptions are to be filled at a local pharmacy, as needed. At study visits, the subject will be asked a Yes/No question whether IR prophylaxis was taken per protocol.

As a precaution, emergency equipment will be readily available to treat a possible hypersensitivity reaction, and will include drugs that would be used to treat an anaphylactic reaction. Personnel trained in managing IRs and in the use of the emergency equipment will be readily available during and for 1 hour after the infusion. As IRs can occur after the completion of the infusion, subjects will be observed for 1 hour post-infusion.

### Infusion Reaction Treatment

Subjects must be monitored closely for signs and symptoms of IRs. In the event of an IR, the infusion should be slowed, or stopped, and restarted at a slower rate at the discretion of the Investigator. If a serious IR occurs, the infusion should be discontinued and treatment should be provided, as needed.

If a subject experiences an AE suspected to be an IR:
A physical examination will be performed to capture medically relevant details, including, but not limited to, a thorough dermatologic examination for detection of erythema, urticaria (hives), or peri-oral or lingual edema; a chest examination for breath sounds, stridor or wheezing; and a cardiac examination with attention to the possibility of an irregular heartbeat;
Vital signs (sitting or supine blood pressure, heart rate, respiratory rate, and body temperature) will be captured at least every 30 minutes until the resolution or stabilization of the AE;
12-lead ECG will be performed; and
A serum sample will be collected in a serum-separating tube at that time or at the subsequent visit. The sample will be centrifuged, frozen at -20°C or colder, and stored for the batch shipment to a Horizon designated laboratory for evaluation of pegloticase antibodies at a future date.

If, in the Investigator's opinion, the subject is experiencing an anaphylactic reaction, pegloticase should be immediately discontinued. Any incidence of anaphylaxis should be reported as an SAE.

The Investigator may administer any medically indicated pharmacologic agent or procedure intended to relieve symptoms (CAUTION: no other drugs can be mixed in the pegloticase infusion bag). Signs and symptoms of the AE and drugs given for treatment will be recorded.

After the first incidence of an IR that does not meet the criteria of anaphylaxis or does not meet serious criteria, the Investigator may elect to initiate the next infusion at a slower rate. Additionally, the Investigator may choose to prescribe 20 mg prednisone to be taken in the morning of the next infusion. All changes to infusion rate or dilution, and drugs given for prophylaxis or treatment, are to be recorded in the medical record and in the eCRF.

### Statistical Methods and Determination of Sample Size

### Populations for Analysis

The following analysis populations will be defined for this study:
Modified intent-to-treat (mITT) population: all subjects who receive at least 1 dose of pegloticase; and
Pharmacokinetic (PK) population: all subjects who receive at least 1 dose of pegloticase and have a post-pegloticase sample evaluable for PK analysis.

The mITT population will be used for analysis of efficacy and safety data.

### Demographic Variables

Demographic data, including age, race, and gender, medical history, and other disease characteristics, will be summarized using descriptive statistics. Listings will include all screened subjects.

### Subject Disposition

The number of subjects in each population and the number and percentage of subjects who complete the study, who discontinue therapy before the Week 22 infusion, and who discontinue the study prematurely along with the reasons for discontinuation will be summarized.

### Efficacy Endpoint Analysis

Efficacy analyses will be performed using the mITT population. No formal statistical testing will be performed, and there is no adjustment for multiplicity in this open-label study. Continuous variables will be summarized using descriptive statistics (number of subjects, mean, median, standard deviation, minimum, and maximum). Categorical variables will be summarized using frequencies and percentages. Unless otherwise specified, baseline is defined as the last non-missing observation prior to the first dose of pegloticase.

### Primary and Secondary Endpoint Analysis

The primary efficacy endpoint is the proportion of responders during Month 6. A responder is defined as a subject for whom the proportion of time that the sUA-time curve is < 6 mg/dL during the analysis interval is at least 80%. The proportion of time that the sUA level is below 6 mg/dL is defined as the ratio of the time during which the sUA level remains below 6 mg/dL (using linear interpolation, if necessary) to the entire time interval during Month 6. The proportion of responders will be summarized, along with a 95% exact (Clopper-Pearson) confidence interval (CI) for the proportion.

A subject will be declared a non-responder if the subject had 2 pre-infusion sUA levels ≥ 6 mg/dL at 2 consecutive study visits beginning with the Week 2 Visit and prior to or during Month 6. Additionally, a subject who withdraws from study treatment for any reason other than the stopping rule after the first dose of pegloticase and prior to or during Month 6 will be considered a non-responder if sUA values are not collected at planned timepoints.

The mean change from baseline to each visit in HAQ pain score and HAQ-DI score will be summarized with descriptive statistics, including 95% CIs for the change from baseline.

Special consideration may be given to subjects who discontinue the study or miss visits due to reasons related to the COVID-19 pandemic.

### Exploratory Endpoint Analysis

HAQ-DI health score, tophus size (longest axis), urate deposition volume, physician global assessment score, mean sUA, eGFR, BP, and UACR will be summarized at baseline and each visit with descriptive statistics. Changes from baseline for these parameters to each visit will be summarized along with 95% CIs.

The proportion of Month 3 responders, overall responders, and 5 mg/dL responders will be analyzed similarly to the primary endpoint. The proportion of subjects with sUA < 6 mg/dL at each visit and the proportion of subjects with gout flares each month will be summarized, along with 95% CIs. The proportion of subjects who experience a gout flare during each month (Month 1 through Month 6) will be based on gout flares reported as adverse events. Gout flares will also be summarized based on the self-reported assessment.

Subjects with resolution of ≥ 1 tophus are subjects with an overall tophus response of complete response (i.e., complete response in at least one tophus and no evidence of progressive disease). The proportion of subjects with resolution of ≥ 1 tophus at Week 24 will be summarized along with the corresponding 95% CI.

### Pharmacokinetic and Anti-drug Antibody Analysis

Concentrations for pegloticase will be summarized using descriptive statistics for the PK population. Incidence of anti-drug antibodies and titer levels will be summarized.

### Safety Analysis

Treatment-emergent AEs are defined as events with an onset date on or after the start of the first pegloticase infusion through 30 days after the last dose of pegloticase. AEs that occur more than 30 days after the last dose of pegloticase through the 3-month follow-up visit will also be summarized.

The number and percentage of subjects experiencing AEs will be summarized by system organ class and preferred term. Summaries by maximum severity and relationship to study drug will also be provided. SAEs and AEs leading to discontinuation of study drug will be presented by system organ class and preferred term. The proportion of subjects with each AESI will also be summarized.

Laboratory test results will be summarized by study visit and change from baseline. Shift tables for laboratory parameters by Common Terminology Criteria for Adverse Events grade will be presented. Laboratory test results will also be classified relative to the normal reference range (normal, low, or high).

Vital signs, including BP, respiratory rate, temperature, and heart rate, will be summarized by study visit and change from baseline.

Prior and concomitant medications will be summarized and/or included in the data listings.

### Interim Analyses

No formal interim analysis is planned for this study. Efficacy and safety data may be summarized after 10-15 subjects have been followed through Week 24. Additional summary of the data may be performed periodically throughout the study, and safety data will be summarized regularly for safety monitoring. Final analysis will occur when all subjects have completed the study.

### Sample Size and Power Considerations

A sample size of 20 subjects is planned for this study. The primary efficacy endpoint, the proportion of subjects achieving and maintaining sUA < 6 mg/dL for at least 80% of the time during Month 6, will be demonstrated to be statistically greater than 43.5% (proportion of responders during Month 6 in the Phase 3 studies), if at least 14/20 (70%) responders are observed. In that case, the lower bound of a 95% confidence interval for the proportion of responders will be about 46%.

### Prior and Concomitant Therapy

Medication history (i.e., prior medications) will include all prior gout medications, starting at the time of diagnosis and up to the Screening Visit, and all other medications taken from 1 year prior to the Screening Visit.

Concomitant medications are defined as drug or biological products other than the study drugs (or prior gout medications) taken by a subject from Screening through the Post-Treatment Follow-up Visits. This includes other prescription medications (including preventive vaccines), over the counter medications, herbal medications, vitamins, and food supplements.

Information about prior and concomitant medications, including those used for any duration to treat an AE, will be collected and recorded at each visit. The generic name of the medication, indication, dose, unit, frequency, route of administration, and start and stop dates will be recorded.

Subjects will be directed to discontinue current ULT prior to initiation of pegloticase therapy as per the current package insert. Other medications used at the time of study initiation may be continued at the discretion of the Investigator.

Subjects should not receive the following medications from the time of Screening through the end of pegloticase treatment: Oral ULTs including allopurinol, febuxostat, probenecid, lesinurad, or other ULT for gout; Any PEG-conjugated drug; nor Any other investigational agent. Re-introduction of oral ULTs should not start until after the End of Pegloticase Visit (or End of Study) laboratory tests are collected.

### Subject Information and Consent

It is the responsibility of the Investigator or a person designated by the Investigator (if acceptable by local regulations) to obtain a signed ICF from each subject prior to participating in this study after adequate explanation of the aims, methods, anticipated benefits, and potential hazards of the study.

The Investigator or designee must also explain that the subjects are completely free to refuse to enter the study or to withdraw from it at any time, for any reason.

The ICF and any other written information provided to subjects will be revised whenever important new information becomes available that may be relevant to the subject's consent, or there is an amendment to the protocol that necessitates a change to the content of the subject information and/or the written ICF. The Investigator will inform the subject of changes in a timely manner and will ask the subject to confirm his/her participation in the study by signing the revised ICF. Any revised written ICF and written information must receive the IRB's approval/favorable opinion in advance of use.

All signed ICFs are to remain in the Investigator's site file or, if locally required, in the subjects' notes/files of the medical institution.

The electronic case report forms (eCRFs) for this study contain a section for documenting all subject ICFs, and this must be completed appropriately. If new safety information results in significant changes in the risk/benefit assessment, the ICF should be reviewed and updated, if necessary. All subjects (including those already being treated) should be informed of the new information, given a copy of the revised ICF, and give their consent to continue in the study.

### Example 2 - Multicenter, Open-Label, Efficacy and Safety Study of Pegloticase in Patients with Uncontrolled Gout who have Undergone Kidney Transplant

Gout's high prevalence in kidney transplant (KT) recipients has been associated with heavy residual urate burden, decreased urate excretion related to reduced renal function, and high calcineurin inhibitor use in this population. The management of gout can be particularly challenging in KT patients due to decreased urate lowering therapy (ULT) renal clearance and drug-drug interactions. Pegloticase (pegylated recombinant uricase) is a treatment indicated for uncontrolled gout and tophi burden that rapidly metabolizes uric acid to allantoin which is readily cleared from the circulation. The use of pegloticase with immunomodulation therapy has been rising in order to reduce anti-drug antibody generation that decreases treatment efficacy and can increase risk of infusion reactions (IRs). Immunomodulator/pegloticase co-therapy markedly improves treatment responder rates. However, only a few case reports of pegloticase use in solid organ transplant patients have been published and phase 3 pegloticase trials excluded organ transplant recipients. The PROTECT trial (NCT04087720) is conducted to examine pegloticase use in renal transplant recipients with uncontrolled gout.

Methods: We are examining pegloticase treatment (12 biweekly 8 mg infusions over 24 weeks) in adult KT recipients (KT >1 year ago, eGFR ≥15 ml/min/1.73m²) with uncontrolled gout (serum uric acid [sUA] ≥ 7 mg/dL, ULT contraindication/inefficacy, and either tophi, chronic gouty arthritis, or ≥ 2 flares in past year). In some embodiments, adult KT recipients may have a sUA of ≥ 6 mg/dL at Screening. The primary endpoint is the proportion of pegloticase responders (sUA < 6 mg/dL for ≥ 80% of time during Month 6). sUA levels, eGFR, urine albumin-to-creatinine ratio (UACR), and health assessment questionnaire (HAQ) pain scores will also be examined.

Results: At the time of this analysis (April 30, 2020), 7 patients who had undergone KT 15.3 ± 5.0 years earlier had enrolled (age: 52.0 ± 11.2 years, sUA: 10.0 ± 1.4 mg/dL, gout duration: 5.9 ± 4.3 years; COVID-19 reduced enrollment). All patients were on stable doses of ≥2 immunosuppressants. 1 patient had completed the 24-week treatment, 5 were in treatment, and 1 had discontinued early due to COVID-19 (non-infection) reasons (Table); the number of infusions received ranged from 2 to 12. In the 5 ongoing patients, all post Day 1 central lab sUA levels were <1 mg/dL, indicative of treatment response. No notable changes in eGFR were observed; 2 patients with baseline albuminuria >300 mg/g showed >35% reduction in UACR by week 14. HAQ pain (0 to 100 VAS) decreased by 33.8 ± 2.7 (n=3) from baseline at week 20. 2 patients experienced flares; no IRs occurred. 2 SAEs (stomach ulcer, cellulitis), deemed unrelated to pegloticase, were reported.

Conclusion: Early findings of the ongoing PROTECT clinical trial suggest that pegloticase can safely and effectively be used in KT recipients to treat uncontrolled/refractory gout. Further efficacy and safety data in the KT population are pending.

**Table. Serum uric acid and kidney function parameters in kidney transplant recipients undergoing pegloticase treatment for uncontrolled gout**

| **Patient** | **sUA (mg/dL)** | | | | **eGFR (ml/min/1.73m²)** | | **UACR** | |
|---|---|---|---|---|---|---|---|---|
| | **Last Visit Prior to Data Cut** | **Baseline** | **Last Value** | **Last Visit with eGFR and UACR values** | **Baseline** | **Last Value** | **Baseline** | **Last Value** |
| 1 | Week 24 | 9.1 | <1 | Week 24 | 61.2 | 51.0 | 376 | 56 |
| 2^{†}* | Week 20 | 7.9 | <1 | Week 14 | 41.6 | 45.5 | 56 | 40 |
| 3* | Week 21 | 9.3 | <1 | Week 14 | 41.8 | 49.7 | 2196 | 1406 |
| 4^{†} | Week 2 | 10.9 | <1 | Week 2 | 41.1 | 34.9 | 305 | 572 |
| 5* | Week 6 | 9.7 | <1 | Week 6 | 40.1 | 40.1 | 22 | 20 |
| 6* | Week 6 | 10.9 | <1 | Week 6 | 40.8 | 32.7 | 317 | 407 |
| 7* | Week 4 | 12.2 | <1 | Week 2 | 20.4 | 23.2 | 407 | 342 |

| | | | | | | | | |
|---|---|---|---|---|---|---|---|---|
| *ongoing pegloticase therapy. ^{†}experienced an SAE (Patient 1, SAE of stomach ulcer deemed unrelated to pegloticase; Patient 4, SAE of cellulitis deemed unrelated to pegloticase). | | | | | | | | |

Aspects and features of the present disclosure are set out in the following numbered clauses which contain the subject matter of the claims of the parent application as filed.
1. A method of treating gout in a kidney transplant recipient having a serum uric acid (SUA) level of ≥ 7 mg/dL comprising administering pegloticase to said kidney transplant recipient at a dose of 8 mg every 2 weeks for a total of 12 doses.
2. The method of clause 1, wherein the kidney transplant recipient is administered gout flare prophylaxis before beginning the pegloticase treatment period.
3. The method of clause 2, wherein the gout flare prophylaxis comprises colchicine and/or prednisone.
4. The method of clause 3, wherein the colchicine is administered at a dose of 0.3 to 0.6 mg/day, and the dose of prednisone is administered at a dose of ≤ 10 mg/day.
5. The method of clause 3, wherein colchicine is administered at a dose of 0.3 mg/day for patients with an estimated glomerular filtration rate (eGFR) of ≤ 30 mL/min/1.73m².
6. The method of clause 3, wherein prednisone is administered at a dose of ≤ 10 mg/day for at least one week before the first pegloticase treatment in subjects taking cyclosporine.
7. The method of clause 3, wherein the gout flare prophylaxis is maintained throughout the pegloticase treatment period.
8. The method of clause 1, further comprising pre-treatment infusion reaction prophylaxis comprising an antihistamine, acetaminophen, and a corticosteroid prior to each treatment with pegloticase.
9. The method of clause 8, wherein the pre-treatment prophylaxis comprises:
   fexofenadine administered at a dose of 60 or 180 mg orally, taken the day before each treatment with PEGylated uricase;
   fexofenadine administered at a dose of 60 or 180 mg orally, and acetaminophen administered at a dose of 1000 mg orally, taken the morning of each treatment with pegloticase ; and
   methylprednisolone administered at a dose of 125 mg intravenously over a methylprednisolone infusion duration between 10 and 30 minutes, immediately prior to each treatment with pegloticase.
10. The method of clause 9, wherein the dose of fexofenadine administered is 60 mg orally in subjects with decreased renal function and 180 mg orally in subjects with normal renal function.
11. The method of clause 10, wherein normal renal function is defined as an estimated glomerular filtration rate (eGFR) of >90 mL/min/1.73m².
12. The method of clause 1, wherein the kidney transplant recipient is taking one or more immunosuppressive agents to prevent rejection of the transplanted kidney.
13. The method of clause 12, wherein the one or more immunosuppressive agents prevents the formation of anti-drug antibodies against pegloticase.
14. The method of clause 13, wherein the prevention of formation of anti-drug antibodies against pegloticase preserves the effectiveness of the pegloticase and decreases the risk of infusion reactions.
15. The method of clause 1, further comprising measuring one or more of hematology, clinical chemistry, urine analysis, pharmacokinetics of pegloticase, and anti-pegloticase antibody during treatment.
16. The method of clause 15, wherein measuring hematology, clinical chemistry, urine analysis, and anti-pegloticase antibody comprises measuring one or more of serum uric acid (SUA) levels, eGFR, urine albumin-to-creatinine ratio (UACR), anti-uricase IgG antibodies, and anti-monomethoxy-poly(ethyleneglycol) (PEG) antibodies.
17. The method of clause 16, wherein the SUA levels of the kidney transplant recipient are determined prior to each dose of the pegloticase.
18. The method of any of clauses 1 to 17, further comprising measuring one or more of trough pegloticase levels, anti-PEGylated uricase antibody levels, and anti-PEG antibody levels, prior to each dose of the pegloticase after the first dose.
19. The method of any of clauses 1 to 18, wherein the SUA level is reduced to less than 6 mg/dL and kidney function is preserved or improved as a result of administration of the pegloticase.
20. The method of any of clauses 1 to 19, wherein the SUA level is reduced to less than 5 mg/dL as a result of administration of the pegloticase.
21. The method of any of clauses 1 to 20, further comprising measuring one or more of peripheral joint urate deposition volume or bone erosion damage.
22. The method of clauses 21, wherein the peripheral joint urate deposition volume or bone erosion damage is measured using Dual Energy Computed Tomography (DECT) scan or any advanced imaging technique.
23. The method of any of clauses 1 to 22, further comprising measuring reduction in tophus size by digital photography.
24. The method of any of clauses 1 to 23, further comprising evaluating pain assessed by the Health Assessment Questionnaire (HAQ) pain score.
25. The method of any of clauses 1 to 24, further comprising evaluating the effect of pegloticase on disability assessed by the Health Assessment Questionnaire - Disability Index (HAQ-DI) score.
26. The method of any of clauses 1 to 25, further comprising evaluating subject-reported changes in physical function and quality of life (QOL).
27. The method of any of clauses 1 to 26, further comprising measuring blood pressure.

## Claims

1. Pegloticase for use in a method of treating gout in a kidney transplant recipient having a serum uric acid (SUA) level of ≥ 7 mg/dL wherein the method comprises administering pegloticase to said kidney transplant recipient at a dose of 8 mg every 2 weeks for a total of 12 doses, and wherein subjects with 2 consecutive measurements of pre-infusion SUA levels greater than 6 mg/dL are discontinued from pegloticase treatment.

2. The pegloticase for use of claim 1, wherein the kidney transplant recipient is administered a gout flare prophylactic before the pegloticase administration.

3. The pegloticase for use of claim 2, wherein the gout flare prophylactic comprises colchicine and/or prednisone.

4. The pegloticase for use of claim 3, wherein the colchicine is administered:
(a) at a dose of 0.3 to 0.6 mg/day, and the dose of prednisone is administered at a dose of ≤ 10 mg/day; or
(b) at a dose of 0.3 mg/day for kidney transplant recipients with an estimated glomerular filtration rate (eGFR) of ≤ 30 mL/min/1.73m².

5. The pegloticase for use of claim 3, wherein the gout flare prophylactic comprises prednisone, and wherein the prednisone is administered at a dose of ≤ 10 mg/day for at least one week before the first pegloticase treatment in kidney transplant recipients taking cyclosporine.

6. The pegloticase for use of claim 3, wherein the gout flare prophylactic is maintained throughout the administration of pegloticase.

7. The pegloticase for use of claim 1, further comprising pre-treatment infusion reaction prophylaxis comprising administering an antihistamine, acetaminophen, and a corticosteroid prior to each treatment with pegloticase.

8. The pegloticase for use of claim 7, wherein the antihistamine is fexofenadine, and wherein:
(a) the fexofenadine is administered at a dose of 60 or 180 mg orally, taken the day before each treatment with pegloticase;
(b) the fexofenadine is administered at a dose of 60 or 180 mg orally, and the acetaminophen is administered at a dose of 1000 mg orally, taken the morning of each treatment with pegloticase; and
(c) the corticosteroid is methylprednisolone, and wherein the methylprednisolone is administered at a dose of 125 mg intravenously over a methylprednisolone infusion duration between 10 and 30 minutes, immediately prior to each treatment with pegloticase.

9. The pegloticase for use of claim 8, wherein the dose of fexofenadine administered is 60 mg orally in kidney transplant recipients with decreased renal function and 180 mg orally in kidney transplant recipients with normal renal function, optionally wherein normal renal function is defined as an estimated glomerular filtration rate (eGFR) of >90 mL/min/1.73m².

10. The pegloticase for use of claim 1, wherein the kidney transplant recipient is taking one or more immunosuppressive agents to prevent rejection of the transplanted kidney, optionally wherein the one or more immunosuppressive agents prevents the formation of anti-drug antibodies against pegloticase.

11. The pegloticase for use of claim 1, further comprising measuring one or more of hematology, clinical chemistry, urine analysis, pharmacokinetics of pegloticase, and anti-pegloticase antibody during treatment, optionally wherein measuring hematology, clinical chemistry, urine analysis, and anti-pegloticase antibody comprises measuring one or more of serum uric acid (SUA) levels, eGFR, urine albumin-to-creatinine ratio (UACR), anti-uricase IgG antibodies, and anti-monomethoxy-poly(ethyleneglycol) (PEG) antibodies, optionally wherein the SUA levels of the kidney transplant recipient are determined prior to each dose of the pegloticase.

12. The pegloticase for use of any of claims 1 to 11, further comprising measuring one or more of trough pegloticase levels, anti-PEGylated uricase antibody levels, and anti-PEG antibody levels, prior to each dose of the pegloticase after the first dose.

13. The pegloticase for use of any of claims 1 to 12, further comprising measuring one or more of peripheral joint urate deposition volume or bone erosion damage, optionally wherein the peripheral joint urate deposition volume or bone erosion damage is measured using Dual Energy Computed Tomography (DECT) scan.

14. The pegloticase for use of any of claims 1 to 13, further comprising:
(a) measuring reduction in tophus size by digital photography;
(b)evaluating pain assessed by the Health Assessment Questionnaire (HAQ) pain score;
(c) evaluating the effect of pegloticase on disability assessed by the Health Assessment Questionnaire - Disability Index (HAQ-DI) score; and/or
(d)evaluating subject-reported changes in physical function and quality of life.

15. The pegloticase for use of claim 2, wherein the kidney transplant recipient has refractory gout.
